# EUROPÄISCHE PATENTSCHRIFT

(11) **EP 4 568 626 B1**
(45) Veröffentlichungstag und Bekanntmachung des Hinweises auf die Patenterteilung: **08.04.2026**
(21) Anmeldenummer: 23758528.6
(22) Anmeldetag: 11.08.2023
(51) Int. Cl.: A61F 13/05, A61F 13/00, A61M 1/00

(54) **VERFAHREN ZUR HERSTELLUNG VON DRAINAGEFOLIEN, SCHLAUCHFÖRMIGE DRAINAGEFOLIE UND WUNDVERSORGUNGSKIT**
METHOD FOR PRODUCING DRAINAGE FILMS, TUBULAR DRAINAGE FILM AND WOUND CARE KIT
PROCÉDÉ DE PRODUCTION DE FILMS DE DRAINAGE, FILM DE DRAINAGE TUBULAIRE ET TROUSSE DE SOINS DE PLAIE

(30) Priorität: 12.08.2022 DE 102022120423
(43) Veröffentlichungstag der Anmeldung: 18.06.2025
(73) Patentinhaber: Lohmann & Rauscher GmbH, 2525 Schönau/Triesting (AT)
(72) Erfinder: LOSKE, Gunnar, 22926 Ahrensburg (DE); NEMEC, Matthias, 2542 Kottingbrunn (AT); ROHRER, Christian, 9500 Villach (AT); STEINLECHNER, Erik, 2500 Baden (AT); GRILLITSCH, Peter, 1230 Wien (AT)
(74) Vertreter: Herrmann, Daniel
(86) Internationale Anmeldenummer: PCT/EP2023/072265
(87) Internationale Veröffentlichungsnummer: WO 2024/033514

(56) Entgegenhaltungen:
- EP-B1- 2 424 477
- US-A1- 2022 152 287

## Beschreibung

Die Erfindung betrifft ein Verfahren zum Herstellen einer doppelwandigen Drainagefolie mit einem offenen Drainageraum und Verfahren zum Herstellen einer schlauchförmigen Drainagefolie. Die Erfindung betrifft darüber hinaus eine schlauchförmige Drainagefolie, ein Wundversorgungskit, sowie die schlauchförmige Drainagefolie bzw. das Wundversorgungskit zur Verwendung bei einer Wundversorgung.

Drainagefolien werden bei verschiedenartigen Krankheitsbildern bzw. Traumata, insbesondere während oder nach Operationen in der Bauchhöhle, Brusthöhle oder Beckenhöhle benötigt, beispielsweise wenn eine temporäre Abdeckung der offenen Wunde und/oder eine Drainage von Körperflüssigkeiten aus der Wundregion notwendig ist. Eine Abdeckung von offenen Wunden kann beispielsweise notwendig sein, wenn mehrere Eingriffe täglich erforderlich sind, um einerseits einen schnellen Zugang zu den inneren Organen zu ermöglichen und andererseits einen nachteilhaften Einfluss der Exsudatbildung im Wundbereich zu vermindern. Durch eine temporäre Abdeckung kann ein deutlicher Rückgang der Mortalität bei einigen Indikationen erreicht werden. Ferner kann eine Absaugung, insbesondere eine postoperative Drainage, von Körperflüssigkeiten erforderlich sein, um die Wundheilung zu begünstigen, da Flüssigkeitsansammlungen wie Blut oder Wundsekret in Wunden den Heilungsprozess stören können.

An für die genannten Einsatzzwecke geeignete Drainagefolie werden grundsätzlich aus medizinischer Sicht zwei Hauptanforderungen gestellt. Zum einen muss ein gutes Exsudatmanagement im Wundbereich, insbes. bei Einsatz in der offenen Bauchhöhle, Brusthöhle oder Beckenhöhle, erreicht werden, d. h. eine Absaugung von Körperflüssigkeiten im gesamten Wundbereich. Darüber hinaus sollte eine Friktionsminderung zwischen der Wunde beziehungsweise umgebenden Organen und der Drainagefolie erreicht werden, während die Wunde gleichzeitig ausreichend von der Umwelt abgeschirmt ist. Darüber hinaus muss sichergestellt werden, dass durch die Drainagefolie keine Verunreinigungen in die offene Wunde bzw. den Körper des Patienten gelangen.

In der EP 0 261 167 B1 ist eine flüssigkeitsdurchlässige und für den direkten Kontakt mit dem Wundgrund vorgesehene Wundabdeckung beschrieben, welche eine hydrophobe Schicht aufweist, um das Anhaften der Wundabdeckung am Wundbereich und eine dadurch ggf. verursachte Verunreinigung der Wunde zu verhindern. Mit der in dieser Schrift beschriebenen Wundabdeckung kann jedoch kein zufriedenstellendes Exsudatmanagement im Wundbereich erhalten werden.

Zur Verbesserung des Exsudatmanagements im Wundbereich wird in der US 7,381,859 B2 eine Wundabdeckung vorgeschlagen, bei der zwischen zwei flüssigkeitsdurchlässigen, folienartigen, bahnförmigen Elementen, die in Form von Kunststofffilmen ausgeführt sein können, eine schaumartige Schicht aufgenommen ist, in der Exsudate absorbiert werden können. Bei der aus dieser Schrift bekannten Wundabdeckung hat es sich jedoch als problematisch erwiesen, dass in Randbereichen der Wundabdeckung keine zufriedenstellende Absaugung von Exsudaten erfolgen kann, so dass es in diesen Randbereichen zu Komplikationen bei der Wundversorgung kommt.

In der WO 2007/118652 A1 ist ein Wunddistanzgitter beschrieben, auf das saugfähige Sekundärverbände kleblos auflegbar sind und welches zur Bildung einer ersten glatten Oberfläche und einer zweiten Oberfläche mit einem rauhen Griff mit einer Vielzahl von dreidimensionalen Perforationen ausgestattet ist. Bei Einsatz dieser bekannten Wundabdeckung sind die nachgeschalteten Absorptionskörper auswechselbar, um so über einen längeren Zeitraum eine zufriedenstellende Wundversorgung zu gewährleisten. Allerdings hat es sich auch bei Einsatz der in der genannten Schrift beschriebenen Wundabdeckungssysteme als problematisch erwiesen, dass ein zufriedenstellendes Exsudatmanagement über den gesamten Wundbereich, insbes. bei tief im Körperinneren liegenden Wunden, nicht erreichbar ist.

In der EP 2 424 477 B1 ist eine doppelwandige Folie zur Wundabdeckung beschrieben, welche einen offenen Drainageraum aufweist. Bei der aus dieser Schrift bekannten Wundabdeckung hat es sich jedoch als problematisch erwiesen, dass bei komplexen Wundregionen, insbesondere tief im Körper liegenden Wunden, keine zufriedenstellende Abdeckung der Wundregionen erzielt werden kann. Ferner hat sich die Herstellung der bekannten Wundabdeckung in Bezug auf die Herstellungseffizienz als problematisch erwiesen.

In der US 2022/0152287 A1 ist eine Wundversorgungsanordnung mit einer mehrlagigen Drainagefolie und einer fluiddichten Befestigungsfolie offenbart.

Angesichts der vorstehend beschriebenen Probleme im Stand der Technik liegt der Erfindung die Aufgabe zugrunde, eine Drainagefolie bereitzustellen, mit der ohne Verunreinigung der Wunde ein gutes Exsudatmanagement über den gesamten Wundbereich, insbesondere auch bei komplexen Wunden, beispielsweise Operationswunden der inneren Organe und/oder des Bewegungsapparates, möglich ist. Darüber hinaus liegt der Erfindung die Aufgabe zugrunde, verbesserte Verfahren zum Herstellen von Drainagefolien bereitzustellen.

### Verfahren zum Herstellen einer doppelwandigen Drainagefolie

Erfindungsgemäß wird diese Aufgabe durch die im Anspruch 1 angegebene Weiterbildung bekannter Verfahren und eine schlauchförmige Drainagefolie gemäß Patentanspruch 15 gelöst.

Bei dem erfindungsgemäßen Verfahren wird durch das Verbinden des ersten bahnförmigen Elements und des zweiten bahnförmigen Elements und der Perforation der bahnförmigen Elemente ein offener Drainageraum gebildet, der eine kapillare Wirkung zwischen den einzelnen bahnförmigen Elementen ermöglicht, mit der einerseits ein Abfließen von Körperflüssigkeiten, insbes. Exsudaten, aus dem Drainageraum verhindert und andererseits eine Verteilung der Körperflüssigkeiten über die gesamte Drainagefläche möglich ist, ohne dass dazu zusätzliche Maßnahmen, wie etwa die Bereitstellung zusätzlicher Absorptionskörper, zwingend erforderlich sind. Auf diese Weise wird ein gutes Exsudatmanagement über den gesamten Wundbereich ermöglicht, weil auch keine das Exsudatmanagement nachteilhaft beeinflussenden Randbereiche ohne Kapillarfunktion vorhanden sind. Das erfindungsgemäße Verfahren ermöglicht ein Herstellen von Drainagefolien, die sich problemfrei auf jede Größe bzw. jede Struktur von Wunden, sogar zur endoluminalen Anwendung, zuschneiden lassen und darüber hinaus über die gesamte Wundfläche die gewünschte Drainagefunktion gewährleisten. Dabei kann das Verfahren umfassen, dass die Drainagefolie mit einer glatten Oberfläche ausgeführt wird, die ein Anhaften von Geweben und Zellwundgrund bzw. bei den umliegenden Organen verhindert. Ferner kann das Verfahren umfassen, dass der Eintritt von Körperflüssigkeiten in den Drainageraum der Drainagefolie dadurch begünstigt wird, dass die bahnförmigen Elemente selbst aus einem flüssigkeitsdurchlässigen Material gebildet werden.

Dabei kann die Stabilität der durch das erfindungsgemäße Verfahren hergestellten Drainagefolien verbessert werden, wenn die mindestens eine den Durchtritt von Körperflüssigkeiten ermöglichende Öffnung durch einen sich ausgehend von dem ersten bzw. zweiten bahnförmigen Element in Richtung des anderen bahnförmigen Elements erstreckenden und in den Drainageraum mündenden Kanal gebildet ist, dessen Kanalwand einstückig mit dem jeweiligen bahnförmigen Element durch Perforation des bahnförmigen Elements gebildet ist.

Bei der zuletzt beschriebenen Ausführungsform der Erfindung kann das Exsudatmanagement besonders wirkungsvoll verbessert werden, wenn die Öffnung bzw. der Kanal derart bereitgestellt wird, dass sich die Querschnittsfläche der Öffnung bzw. des Kanals in einer sich senkrecht zu einer Tiefenrichtung der Drainagefolie erstreckenden Ebene ausgehend von dem einen bahnförmigen Element in Richtung auf das andere bahnförmige Element, insbes. zum Erhalt einer den Eintritt von Körperflüssigkeiten in den Drainageraum begünstigenden kapillaren Wirkung, verringert. Auf diese Art und Weise wird einerseits der Abtransport von Exsudat aus dem Wundbereich unterstützt und andererseits ein Zurückfließen aus dem Drainageraum in den Wundraum verhindert.

Im Rahmen der Erfindung hat es sich in Bezug auf ein effektives Exsudatmanagement als besonders günstig erwiesen, wenn die mindestens eine den Durchtritt von Körperflüssigkeiten ermöglichende Öffnung einen Durchmesser in einem Bereich von 100 µm bis 2000 µm, bevorzugt in einem Bereich von 300 µm bis 700 µm, bevorzugter in einem Bereich von 400 µm bis 600 µm, aufweist. Es hat sich herausgestellt, dass durch mindestens eine, bevorzugt eine Vielzahl von den Durchtritt von Körperflüssigkeiten ermöglichende(n) Öffnung(en) mit einem solchen Durchmesser eine hervorragende kapillare Wirkung erzielt werden kann.

Es hat sich darüber hinaus herausgestellt, dass ein besonders gutes Exsudatmanagement und eine besonders gute Stabilität der Drainagefolie dann gewährleistet werden, wenn das erste bahnförmige Element und das zweite bahnförmige Element an einer Vielzahl von Befestigungsbereichen, bevorzugt an einer Vielzahl von in einer Rasteranordnung ausgebildeten Befestigungsbereichen, verbunden werden. Im Sinne einer hervorragenden Absaugungsfähigkeit der Drainagefolie beträgt ein Abstand zwischen jeweils benachbarten Befestigungsbereichen bevorzugt 2 mm oder mehr, bevorzugt 3 mm oder mehr, bevorzugter 5 mm oder mehr. Die Vielzahl von Befestigungsbereichen kann beispielsweise in einem Rechteckraster angeordnet sein. Eine Anordnung in einem Rechteckraster ermöglicht eine besonders effiziente Herstellung der Drainagefolie.

Das Verbinden der bahnförmigen Elemente kann unter gleichzeitiger Sicherstellung des die Drainagewirkung hervorbringenden Drainageraums über eine Vielzahl von punktförmigen und vorzugsweise in einer Rasteranordnung ausgebildeten Befestigungsbereichen erfolgen. Das Verbinden kann dabei mittels Schweißen, Kleben oder anderen festen Verbindungsarten geschehen. Dabei sollte durch die Verbindung der Abtransport von Exsudaten jedoch nicht behindert, sondern unterstützt werden. Es hat sich herausgestellt, dass ein Verbinden mittels Ultraschallverschweißen nicht nur eine effizientere Herstellung der Drainagefolie als andere Methoden zum Verbinden der bahnförmigen Elemente ermöglicht, sondern auch besonders stabile Drainagefolien hervorbringt, deren strukturelle Integrität auch unter mechanischer Belastung, zum Beispiel bei einer endoluminalen Anwendung, gewährleistet wird. Insbesondere hat sich herausgestellt, dass ein Verbinden mittels Ultraschallverschweißen ein Herstellen von besonders strapazierfähigen Drainagefolien ermöglicht und eine besonders vorteilhafte Verbundfestigkeit von 0,5 N/25,4mm oder mehr erzielt werden kann.

Es hat sich als günstig erwiesen, wenn die einzelnen Befestigungsbereiche in einer sich senkrecht zur Tiefenrichtung erstreckenden Schnittebene eine Fläche von 5 mm² oder weniger, insbes. 3 mm² oder weniger, besonders bevorzugt 2 mm² oder weniger, aufweisen, wobei der Abstand zwischen einzelnen Befestigungsbereichen bzw. einzelnen Rasterpunkten der in einer Rasteranordnung ausgeführten Befestigungsbereiche 2 mm oder mehr, im Besonderen 3 mm oder mehr, besonders bevorzugt 5 mm oder mehr, beträgt.

Unabhängig davon, ob die Befestigung durch Schweißen, Kleben oder andere Verbindungsarten erfolgt, kann in den Befestigungsbereichen eine die inneren Begrenzungsflächen der bahnförmigen Elemente miteinander verbindende Materialbrücke gebildet werden. Es hat sich herausgestellt, dass eine solche die Stabilität der Gesamtstruktur der Drainagefolie begünstigende Materialbrücke besonders präzise mittels Ultraschallverschweißen hergestellt werden kann. Zum Erhalt der gewünschten Kapillarwirkung im Drainageraum beträgt der Abstand zwischen den inneren Begrenzungsflächen der bahnförmigen Elemente 5 mm oder weniger, vorzugsweise 4 mm oder weniger, besonders bevorzugt 2 mm oder weniger. Dabei kann die Verteilung der Exsudate über die Gesamtfläche der Drainagefolie unter Ausnutzung der Kapillarwirkung im Drainageraum sichergestellt werden, wenn der Abstand zwischen den inneren Begrenzungsflächen der bahnförmigen Elemente 0,05 mm oder mehr, insbes. 0,1 mm oder mehr, besonders bevorzugt 0,3 mm oder mehr, beträgt. Zur Herstellung von hochqualitativen Drainagefolien hat es sich als besonders günstig erwiesen, wenn das Verbinden des ersten bahnförmigen Elements und des zweiten bahnförmigen Elements mittels Ultraschallverschweißen durchgeführt wird, da so der gewünschte Abstand zwischen dem ersten und zweiten bahnförmigen Element, insbesondere zwischen einer von dem ersten bahnförmigen Element gebildeten inneren Begrenzungsfläche des Drainageraums und einer von dem zweiten bahnförmigen Element gebildeten inneren Begrenzungsfläche des Drainageraums, präzise und qualitätssichernd hergestellt werden kann. Es hat sich herausgestellt, dass eine Herstellung der Drainagefolie mittels Ultraschallverschweißen höherqualitative Drainagefolien ermöglicht, als wenn der Abstand zwischen den inneren Begrenzungsflächen nur durch die Tiefe von insbesondere trichterförmigen Öffnungen sichergestellt wird, da eine Perforation typischerweise nur mit begrenzter Genauigkeit erfolgt.

Für die Anwendung der Drainagefolie bei der Wundversorgung hat es sich als besonders vorteilhaft erwiesen, wenn die Drainagefolie ein Flächengewicht in einem Bereich von 30 g/m² bis 90 g/m², bevorzugt 40 g/m² bis 80 g/m², bevorzugter 50 g/m² bis 70 g/m², aufweist. Das erste bahnförmige Element und/oder das zweite bahnförmige Element können/kann eine Kunststofffolie umfassen oder sein, wobei die Kunststofffolie bevorzugt Polyethylen, Polypropylen, Polyethylenterephthalat, Polyurethan, Polytetrafluorethylen, Polyhydroxybutyrat, Polylactid und/oder Cellulose umfasst. Solche Kunststofffolien haben den Vorteil, dass sie ein Eindringen von Pathogenen, beispielsweise Bakterien, und ein Anhaften und Einwachsen von Gewebe effektiv verhindern. Die Kunststofffolie kann insbesondere Polyethylen, bevorzugt Low-Density-Polyethylen (PE-LD) umfassen. Es hat sich herausgestellt, dass bahnförmige Elemente bzw. Kunststofffolien mit Polyethylen, insbesondere Low-Density-Polyethylen (PE-LD), in Bezug auf die Herstellung und Anwendung von erfindungsgemäßen Drainagefolien besonders vorteilhaft sind. Das erste bahnförmige Element und/oder das zweite bahnförmige Element können/kann Polyethylen, Polypropylen, Polyethylenterephthalat, Polyurethan, Polytetrafluorethylen, Polyhydroxybutyrat, Polylactid und/oder Cellulose, bevorzugt Polyethylen, bevorzugter Low-Density-Polyethylen (PE-LD), umfassen. Die Struktur der bahnförmigen Elemente kann mittels eines non woven Herstellungsprozesses erzeugt werden. Dabei können zur Herstellung der non wovens oder auch Gewebe synthetische wie auch natürliche (z. B. Seide, Baumwolle) Fasern, wie auch anorganische Fasern (Glaskeramik...) bzw. Metall (Silberfasern) verwendet werden. Im Rahmen der Erfindung ist ferner daran gedacht, dass die bahnförmigen Elemente einseitig oder beidseitig mit einer antibakteriellen bzw. bakteriostatischen Schicht ausgestattet werden. Die antibakterielle bzw. bakteriostatische Wirkung kann bspw. durch PHMB, Silber, Chlorhexidin usw. verwirklicht werden. Im Hinblick auf die Vermeidung der Verklebungsneigung der durch das erfindungsgemäße Verfahren hergestellten Drainagefolien kann mindestens ein bahnförmiges Element eine hydrophile oder hydrophobe Ausrüstung aufweisen. Ferner ist auch an den Auftrag von quellbaren Materialen gedacht.

Es hat sich in Bezug auf die Anwendung der durch das erfindungsgemäße Verfahren hergestellten Drainagefolie bei der Wundversorgung als besonders vorteilhaft herausgestellt, wenn die Drainagefolie eine Zugfestigkeit von 7 N/25,4mm oder mehr, eine Reißdehnung von 40% oder mehr, eine Porosität von 75 m³/m²/min oder mehr, und/oder eine Verbundfestigkeit von 0,5 N/25,4mm oder mehr, aufweist. Ein effektives Exsudatmanagement bei gleichzeitiger Sicherstellung der strukturellen Integrität der Drainagefolie kann so gewährleistet werden. Die Zugfestigkeit wird bevorzugt gemäß DIN EN ISO 527 gemessen. Die Drainagefolie kann beispielsweise eine Zugfestigkeit von 7 N/25,4mm oder mehr, gemessen gemäß DIN EN ISO 527, aufweisen. Die Reißdehnung wird bevorzugt gemäß DIN EN ISO 527 gemessen. Die Drainagefolie kann beispielsweise eine Reißdehnung von 40% oder mehr, gemessen gemäß DIN EN ISO 527, aufweisen. Die Verbundfestigkeit wird bevorzugt in Anlehnung an Edana NWSP 401.0.R0 gemessen. Die Drainagefolie kann beispielsweise eine Verbundfestigkeit von 0,5 N/25,4mm oder mehr, gemessen in Anlehnung an Edana NWSP 401.0.R0 oder gemessen gemäß Edana NWSP 401.0.R0, aufweisen. Die Porosität ist ein Maß für das Verhältnis des Hohlraumvolumens zu dem Gesamtvolumen der Drainagefolie.

Bei nach dem erfindungsgemäßen Verfahren hergestellten Drainagefolien kann die mindestens eine den Durchtritt von Körperflüssigkeiten ermöglichende Öffnung, insbesondere bezogen auf eine Aufsicht auf eine flächige Erstreckung des ersten bzw. zweiten bahnförmigen Elements, eine runde, kreisförmige und/oder ovale Form aufweisen. Eine solche Form ermöglicht ein effektives Hindurchtreten von Körperflüssigkeiten aus der Wundregion in den offenen Drainageraum der Drainagefolie.

Bei den durch das erfindungsgemäße Verfahren hergestellten Drainagefolien kann der gewünschte Abtransport des Exsudats unter Sicherstellung einer zufriedenstellenden Gesamtstabilität der Drainagefolie erreicht werden, wenn mindestens ein bahnförmiges Element, bevorzugt beide bahnförmige Elemente, eine Vielzahl von vorzugsweise rasterförmig angeordneten, insbes. in einem Rechteckraster angeordneten Öffnungen, aufweist/aufweisen, wobei der Abstand zwischen benachbarten Öffnungen bzw. Rasterpunkten eines bahnförmigen Elements 15 mm oder weniger, vorzugsweise 5 mm oder weniger, insbes. 3 mm oder weniger, beträgt. Im Hinblick auf die gewünschte Gesamtstabilität der durch das erfindungsgemäße Verfahren hergestellten Drainagefolien hat es sich weiter als besonders zweckmäßig erwiesen, wenn die Mündungen der in einem der bahnförmigen Elemente angeordneten Öffnungen in einer Projektion längs der Tiefenrichtung zwischen den Mündungen der in dem anderen bahnförmigen Element angeordneten Öffnungen angeordnet sind. Um ein Kollabieren der Gesamtstruktur der Drainagefolie, insbesondere bei Anlegen von Unterdruck, zu verhindern, hat es sich als besonders günstig erwiesen, wenn sich mindestens ein eine Öffnung in einem bahnförmigen Element bildender Kanal, bevorzugt eine Vielzahl von solchen Kanälen, in Tiefenrichtung über 50 % oder mehr der gesamten Tiefe des Drainageraums erstreckt.

Zur Sicherstellung der gewünschten Durchlässigkeit der bahnförmigen Elemente hat es sich als zweckmäßig erwiesen, wenn die Perforation derart durchgeführt wird, dass die dem Drainageraum zugewandte Mündungsfläche der einzelnen Öffnungen in einer senkrecht zur Tiefenrichtung verlaufenden Ebene 0,1 mm² oder mehr, insbes. 0,5 mm² oder mehr, besonders bevorzugt 1 mm² oder mehr, beträgt. Die gewünschte Kapillarwirkung kann unter Vermeidung eines Rückflusses von Flüssigkeit aus dem Drainageraum in den Wundraum erreicht werden, wenn die Perforation derart durchgeführt wird, dass die Mündungsfläche der Öffnungen in der erhaltenen Drainagefolie 5 mm² oder weniger, insbes. 4 mm² oder weniger, besonders bevorzugt 3 mm² oder weniger, beträgt.

Wie vorstehend bereits erläutert, werden die Öffnungen in den bahnförmige Elemente bildenden Kanäle durch Perforationen in den bahnförmigen Elementen gebildet. In diesem Zusammenhang hat es sich zum Erhalt einer glatten Oberfläche, mit der ein Anhaften von Gewebe bzw. Zellen am Wundgrund bzw. bei den umliegenden Organen wirkungsvoll unterdrückt werden kann, als günstig erwiesen, wenn die Kanalwand in einer sich parallel zur Tiefenrichtung erstreckenden Schnittebene zumindest abschnittweise bogenförmig ausgeführt ist und stetig in die Begrenzungsfläche des bahnförmigen Elements übergeht.

Das erfindungsgemäße Verfahren kann ein Anbringen eines Haftmittels, insbesondere eines Klebstoffs, eines adhäsiven Materials und/oder eines Klettverbindungsmittels, insbesondere eines oder mehrerer Widerhaken, an der Drainagefolie umfassen. Solche Haftmittel, adhäsiven Materialien, oder Klettverbindungsmittel ermöglichen, dass die hergestellte Drainagefolie an anderen Strukturen befestigt werden kann, beispielsweise an Körperteilen, Operationsvorrichtungen, Absorptionskörpern, Schläuchen, Drähten, Stents, Einführhilfen, und/oder Vorrichtungen zur Unterdrucktherapie. Beispielsweise kann das Verfahren eine Anbringen des Haftmittels, adhäsiven Materials und/oder Klettverbindungsmittels an zwei gegenüberliegenden Kanten der Drainagefolie umfassen. Eine solche Drainagefolie kann dann dazu verwendet werden, eine Struktur, beispielsweise ausgewählt aus Absorptionskörpern, Schläuchen, Drähten, Stents, Einführhilfen, und Vorrichtungen zur Unterdrucktherapie, zu umwickeln und durch Verbinden des Haftmittels, adhäsiven Materials beziehungsweise Klettverbindungsmittels der zwei gegenüberliegenden Kanten die Drainagefolie an der Struktur zu fixieren.

Herstellungstechnisch hat es sich als besonders vorteilhaft erwiesen, wenn Schritt ii) vor oder nach Schritt iii) durchgeführt wird, und/oder Schritt iv) nach Schritt iii) und vor oder nach Schritt ii) durchgeführt wird.

Das erfindungsgemäße Verfahren kann darüber hinaus ein Bilden einer dreidimensionalen, bevorzugt schlauchförmigen Struktur aus der Drainagefolie umfassen. Der Vorteil solcher dreidimensionalen Strukturen, beispielsweise schlauchförmigen Strukturen, ist, dass diese für komplexere Wunden, beispielsweise tiefe Wunden, bzw. in tiefen Regionen des Körpers eingesetzt werden können. Insbesondere können solche dreidimensionalen Strukturen, wie schlauchförmigen Strukturen, sogar endoluminal, beispielsweise im Gastrointestinaltrakt oder in Fistelgängen, eingesetzt werden. Die Drainagefolie kann schlauchförmig ausgeführt sein, beispielsweise zu einer endoluminalen Anwendung und/oder zum Aufnehmen eines Drainageschlauches.

Herstellungstechnisch hat es sich als besonders günstig erwiesen, wenn eine solche dreidimensionale Struktur durch Schweißen, insbesondere Ultraschallverschweißen oder Laserschweißen, durch thermisches Verbinden, insbesondere mittels UV oder Heißluft, unter Verwendung eines Haftmittels und/oder durch Tiefziehen, insbesondere durch Tiefziehen der in Schritt iv) verbundenen ersten und zweiten bahnförmigen Elemente, gebildet wird.

### Verfahren zum Herstellen einer schlauchförmigen Drainagefolie

Die Aufgabe der Erfindung wird darüber hinaus durch ein erfindungsgemäßes Verfahren zum Herstellen einer schlauchförmigen Drainagefolie gelöst, das dadurch gekennzeichnet ist, dass das Verfahren die folgenden Schritte umfasst:
a) Bereitstellen einer Drainagefolie, bevorzugt einer doppelwandigen Drainagefolie mit einem offenen Drainageraum, bevorzugter einer nach dem erfindungsgemäßen Verfahren zum Herstellen einer doppelwandigen Drainagefolie hergestellten doppelwandigen Drainagefolie;
b) Umklappen, insbesondere Falten der Drainagefolie um eine Klapp-, insbesondere Faltlinie, derart auf sich selbst, dass die Drainagefolie einen zu einer Verbindungslinie verbindbaren Überlappungsbereich aufweist; wobei, bevorzugt, die Drainagefolie eine erste Kante und eine zur ersten Kante im Wesentlichen parallel verlaufende zweite Kante aufweist und die Drainagefolie längs der Klapp- bzw. Faltlinie derart auf sich selbst geklappt bzw. gefaltet wird, dass die Drainagefolie entlang der ersten Kante und der zweiten Kante den zu der Verbindungslinie verbindbaren Überlappungsbereich aufweist;
c) Verbinden der Drainagefolie längs des Überlappungsbereichs, wobei eine Verbindungslinie gebildet wird;
d) Erhalten einer schlauchförmigen Drainagefolie.

Darüber hinaus wird die Aufgabe der Erfindung durch ein erfindungsgemäßes Verfahren zum Herstellen einer schlauchförmigen Drainagefolie gelöst, das dadurch gekennzeichnet ist, dass das Verfahren die folgenden Schritte umfasst:
a) Bereitstellen einer Drainagefolie, bevorzugt einer doppelwandigen Drainagefolie mit einem offenen Drainageraum, bevorzugter einer nach dem erfindungsgemäßen Verfahren zum Herstellen einer doppelwandigen Drainagefolie hergestellten doppelwandigen Drainagefolie;
b) Anordnen einer ersten Kante der Drainagefolie auf dem elastischen Material derart, dass ein zu einer Verbindungslinie verbindbarer Überlappungsbereich zwischen der ersten Kante der Drainagefolie und dem elastischen Material gebildet wird;
c) Verbinden der Drainagefolie und des elastischen Materials längs des Überlappungsbereichs zwischen der ersten Kante der Drainagefolie und dem elastischen Material, wobei eine Verbindungslinie gebildet wird;
d) Umklappen, insbesondere Falten der Drainagefolie bzgl. einer Klapp-, insbesondere einer Faltlinie, derart auf das elastische Material, dass ein zu einer Verbindungslinie verbindbarer Überlappungsbereich zwischen einer zweiten Kante der Drainagefolie, bevorzugt einer zu der ersten Kante im Wesentlichen parallel verlaufenden zweiten Kante der Drainagefolie, und dem elastischen Material gebildet wird;
e) Verbinden der Drainagefolie und des elastischen Materials längs des Überlappungsbereichs zwischen der zweiten Kante der Drainagefolie und dem elastischen Material, wobei eine Verbindungslinie gebildet wird;
f) Erhalten einer schlauchförmigen Drainagefolie.

Die Aufgabe Erfindung wird auch gelöst durch ein erfindungsgemäßes Verfahren zum Herstellen einer schlauchförmigen Drainagefolie, das dadurch gekennzeichnet ist, dass das Verfahren die folgenden Schritte umfasst:
a) Bereitstellen einer Drainagefolie, bevorzugt einer doppelwandigen Drainagefolie mit einem offenen Drainageraum, bevorzugter einer nach dem erfindungsgemäßen Verfahren zum Herstellen einer doppelwandigen Drainagefolie hergestellten doppelwandigen Drainagefolie;
b) Wickeln der Drainagefolie um eine Wickelachse derart, dass die Drainagefolie einen durch das Wickeln um die Wickelachse gebildeten, zu einer Verbindungslinie verbindbaren Überlappungsbereich aufweist, in dem die Drainagefolie mindestens doppellagig vorliegt;
c) Verbinden einer ersten Lage und einer zweiten Lage der in dem Überlappungsbereich mindestens doppellagig vorliegenden Drainagefolie, bevorzugt durch ein Haftmittel oder ein Verbindungsmittel; wobei, bevorzugt, eine Verbindungslinie gebildet wird;
d) Erhalten einer schlauchförmigen Drainagefolie.

Es hat sich herausgestellt, dass die vorangehend genannten erfindungsgemäßen Verfahren zum Herstellen einer schlauchförmigen Drainagefolie jeweils effizient eine schlauchförmige Drainagefolie bereitstellen, die eine gleichmäßige Drainageleistung sogar in komplexen Wundregionen, beispielsweise bei tief im Körper liegenden Wunden, ermöglicht. Die nachfolgenden Ausführungen beziehen sich auf alle drei vorangehend genannten erfindungsgemäßen Verfahren zum Herstellen einer schlauchförmigen Drainagefolie. Jedes der vorangehend genannten Verfahren zum Herstellen einer schlauchförmigen Drainagefolie kann die nachfolgenden Eigenschaften aufweisen.

Herstellungstechnisch hat es sich als besonders vorteilhaft erwiesen, wenn das Verbinden durch Schweißen, insbesondere Ultraschallverschweißen oder Laserschweißen, durch thermisches Verbinden, insbesondere mittels UV oder Heißluft, durch ein Haftmittel, insbesondere einen Klebstoff oder ein Klettverbindungsmittel, und/oder durch ein Verbindungsmittel, insbesondere eine Klammer, durchgeführt wird. Insbesondere kann das Verbinden der Drainagefolie längs des Überlappungsbereichs, das Verbinden der Drainagefolie und des elastischen Materials längs des Überlappungsbereichs zwischen der ersten Kante der Drainagefolie und dem elastischen Material, das Verbinden der Drainagefolie und des elastischen Materials längs des Überlappungsbereichs zwischen der zweiten Kante der Drainagefolie und dem elastischen Material, sowie das Verbinden einer ersten Lage und einer zweiten Lage der in dem Überlappungsbereich mindestens doppellagig vorliegenden Drainagefolie durch Schweißen, insbesondere Ultraschallverschweißen oder Laserschweißen, durch thermisches Verbinden, insbesondere mittels UV oder Heißluft, durch ein Haftmittel, insbesondere einen Klebstoff oder ein Klettverbindungsmittel, und/oder durch ein Verbindungsmittel, insbesondere eine Klammer, durchgeführt werden.

Ein Verbindungsmittel kann beispielsweise eine Klammer, eine Schnur, ein Faden, eine Naht, beispielsweise eine elastische Naht beziehungsweise eine Naht mit einem elastischen Material, oder eine ringförmige Applikationshilfe sein. Beispielsweise kann eine Drainagefolie um eine Wickelachse gewickelt werden, insbesondere um einen Drainageschlauches gewickelt werden, und dann mittels eines Verbindungsmittels, beispielsweise einer Klammer oder ringförmigen Applikationshilfe als schlauchförmige Drainagefolie fixiert werden. Ein Überlappungsbereich, beispielsweise ein Überlappungsbereich zwischen einer ersten und zweiten Kante einer doppelwandigen Drainagefolie, kann mittels einer elastischen Naht verbunden werden. Eine elastische Naht steigert die Aufdehnbarkeit der schlauchförmigen Drainagefolie.

Die hergestellten schlauchförmigen Drainagefolien haben für die medizinische Anwendung besonders vorteilhafte Eigenschaften, wenn das Verbinden durch Ultraschallverschweißen durchgeführt wird, bevorzugt mit einer Geschwindigkeit in einem Bereich von 0,1 bis 5,0 m/min, einer Leistung in einem Bereich von 50 bis 500 Watt und/oder einem Druck in einem Bereich von 5N bis 100N. Ein solches Verbinden ermöglicht eine stabile Verbindungslinie, die selbst unter mechanischer Belastung eine hohe Reißfestigkeit gewährleistet.

Das Verbinden in den erfindungsgemäßen Verfahren zum Herstellen einer schlauchförmigen Drainagefolie, insbesondere das Verbinden der Drainagefolie längs des Überlappungsbereichs, das Verbinden der Drainagefolie und des elastischen Materials längs des Überlappungsbereichs zwischen der ersten Kante der Drainagefolie und dem elastischen Material, das Verbinden der Drainagefolie und des elastischen Materials längs des Überlappungsbereichs zwischen der zweiten Kante der Drainagefolie und dem elastischen Material, und/oder das Verbinden einer ersten Lage und einer zweiten Lage der in dem Überlappungsbereich mindestens doppellagig vorliegenden Drainagefolie kann ein Anordnen der Drainagefolie in einer Ultraschallverschweißungsvorrichtung und Verschweißen der Drainagefolie, insbesondere mittels einer Schweißform, z.B. einer L-förmigen Schweißform, zu einer schlauchförmigen Drainagefolie, umfassen. Es hat sich herausgestellt, dass schlauchförmige Drainagefolien mit entsprechenden Schweißformen effizient und in hoher Qualität hergestellt werden können.

Es hat sich darüber hinaus herausgestellt, dass die Eigenschaften der Schnittkante für die Funktionalität der Drainagefolie dann besonders geeignet sind, wenn das Verbinden durch Ultraschallverschweißen unter Verwendung eines Schneidrades mit einem Schneidkantenradius von 0,2 mm oder weniger, und/oder unter Verwendung eines Schneidrades mit einem der Klapp-, insbesondere Faltlinie zugewandten Schleifwinkel von 15° oder weniger und/oder mit einem der Klapp-, insbesondere Faltlinie abgewandten Schleifwinkel von 75° oder weniger, durchgeführt wird. So kann verhindert werden, dass Materialreste, welche die Funktionalität der Drainagefolie beeinträchtigen könnten, an der Schnittkante verbleiben.

Ein Verbinden mittels Schweißen, insbesondere Ultraschallverschweißen, kann beispielsweise dadurch durchgeführt werden, dass das Material im Überlappungsbereich, beispielsweise die doppelwandige Drainagefolie und/oder ein elastisches Material, durch die vom Generator zum Schwingen gebrachte Sonotrode erwärmt und so verschmolzen wird. Um eine Schweißnaht herzustellen, kann ein Schneidrad verwendet werden, beispielsweise ein Schneidrad, welches gleichzeitig schweißen und schneiden kann. Durch ein Schneidrad, welches gleichzeitig schweißen und schneiden kann, entsteht eine Schweißnaht, bei der das überschüssige Material während dem Schweißprozess gleichzeitig entfernt wird. Es kann auch ein herkömmliches Schneidrad verwendet werden, um eine Verbindungslinie zu erzeugen. Ein Ultraschallverschweißen kann beispielsweise mit einer Nucleus Rotosonic DX1 - TC 12 Flatbed Ultraschallverschweißvorrichtung durchgeführt werden, beispielsweise mit einer Ultraschallfrequenz von etwa 35kHz.

Um eine möglichst glatte Verbindungslinie bereitzustellen, insbesondere um durch eine raue Verbindungslinie etwaig eintretende Funktionsbeeinträchtigungen der schlauchförmigen Drainagefolie zu verhindern, kann ein auf einer der Klapp-, insbesondere Faltlinie abgewandten Seite der Verbindungslinie angeordneter Randstreifen der Drainagefolie während des Verbindens oder nach dem Verbinden, insbesondere durch Stanzen, entfernt werden. Darüber hinaus kann ein Stanzen dazu verwendet werden, um restliches Material, welches nicht benötigt wird, zu entfernen.

Es hat sich herausgestellt, dass es für die Funktionalität der schlauchförmige Drainagefolie besonders vorteilhaft ist, wenn das Verfahren so durchgeführt wird, dass die Verbindungslinie, beispielsweise Schweißnaht, entlang einer Umfangsrichtung der schlauchförmigen Drainagefolie eine Breite von 2 mm oder weniger, bevorzugt 1 mm oder weniger, bevorzugter 0,5 mm oder weniger, aufweist. Um etwaige Funktionsbeeinträchtigung des Produktes zu verhindern, ist es besonders vorteilhaft, wenn eine möglichst schmale Verbindungslinie, insbesondere Schweißnaht, bereitgestellt wird. Darüber hinaus hat es sich für eine Gewährleistung der Funktionalität im gesamten Bereich der Schlauchstruktur als besonders günstig erwiesen, wenn die Verbindungslinie entlang einer, insbesondere sich senkrecht zur Schlauchachse erstreckenden, Radialrichtung der schlauchförmigen Drainagefolie beziehungsweise Tiefenrichtung eine Dicke von 1 mm oder weniger, bevorzugt 750 µm oder weniger, bevorzugter 450 µm oder weniger, aufweist. Je nachdem auf welche Weise das Verbinden bei den erfindungsgemäßen Verfahren zum Herstellen von schlauchförmigen Drainagefolien durchgeführt wird, kann das Material im Überlappungsbereich, insbesondere an der Verbindungslinie, verschmelzen und so eine Materialbrücke bilden, beispielsweise wenn das Verbinden mittels Schweißen, insbesondere Ultraschallverschweißen, durchgeführt wird.

Erfindungsgemäß kann das Verfahren zum Steigern der Stabilität der Drainagefolie ein Anbringen eines Verstärkungselements, insbesondere eines Verstärkungsstreifens oder einer Verstärkungsfolie, in einem Bereich der Verbindungslinie, bevorzugt durch Anordnen des Verstärkungselements im Überlappungsbereich vor dem Verbinden, und/oder an mindestens einem Schlauchende, umfassen. Ein Verstärkungsstreifen kann beispielsweise dadurch bereitgestellt werden, dass die Drainagefolie im zu verstärkenden Bereich doppellagig bereitgestellt wird oder ein verstärkendes Material, beispielsweise eine Polyethylen-Folie, im zu verstärkenden Bereich befestigt wird. Es hat sich herausgestellt, dass eine Folie, insbesondere eine Polyethylen-Folie, als Verstärkungselement einen besonders vorteilhaften Verstärkungseffekt hat.

Beispielweise kann das Verstärkungselement bei dem Verbinden zusätzlich mitverschweißt werden, indem das Verstärkungselement vor dem Verbinden im Überlappungsbereich angeordnet wird, um so die Reißkraft der Drainagefolie, insbesondere die Reißkraft an der Verbindungslinie, zu erhöhen. Das Verstärkungselement hat nicht nur den Vorteil, dass es die Reißkraft der Drainagefolie erhöht, sondern kann auch weitere Vorteile bieten, zum Beispiel als ein röntgenkontrastfähiges Verstärkungselement ausgeführt sein. Dies ermöglicht beispielsweise eine präzise Platzierung der schlauchförmigen Drainagefolie an der Zielstruktur unter Kontrolle von Röntgenbildgebung.

Die hervorragende strukturelle Integrität der schlauchförmigen Drainagefolie kann darüber hinaus auch weiter gesteigert werden, wenn die schlauchförmige Drainagefolie an mindestens einem Schlauchende in einer Umfangsrichtung mit einem Verstärkungsbereich, in dem die Drainagefolie mindestens doppellagig vorliegt, ausgeführt wird. Dies ist vor allem für solche Anwendungen vorteilhaft, bei denen Hilfsmittel, beispielsweise ein Absorptionskörper, Schlauch, Draht, Stent oder eine Einführhilfe, in die schlauchförmige Drainagefolie eingeschoben werden sollen, beispielsweise während chirurgischen Operationen oder bei der Herstellung von Wundversorgungskits. Durch den Verstärkungsbereich wird die Schlauchöffnung weiter stabilisiert, sodass beliebige Hilfsmittel in die schlauchförmige Drainagefolie eingeführt werden können, ohne dass es zu einer Gefahr des Einreißens der Drainagefolie kommt. Herstellungstechnisch hat es sich als vorteilhaft erwiesen, wenn der Verstärkungsbereich durch ein Umklappen eines Abschnitts der schlauchförmigen Drainagefolie am Schlauchende, insbesondere eines Randbereichs der schlauchförmigen Drainagefolie, gebildet wird, wobei das Umklappen insbesondere derart durchgeführt wird, dass ein die innere Begrenzungsfläche der schlauchförmigen Drainagefolie bildendes schlauchförmiges Element im Verstärkungsbereich die äußere Begrenzungsfläche der schlauchförmigen Drainagefolie bildet. Das Verfahren kann ferner ein Fixieren, beispielsweise ein punktuelles Fixieren, des umgeklappten Abschnitts der schlauchförmigen Drainagefolie umfassen, insbesondere durch Schweißen, thermisches Verbinden, ein Haftmittel, und/oder ein Verbindungsmittel. Als "Umklappen" ist jedes Formen, Anordnen, Verbiegen, Wickeln, bzw. Falten einer Drainagefolie zu verstehen, bei dem ein Überlappungsbereich, insbesondere ein zu einer Verbindungslinie verbindbarer Überlappungsbereich, und/oder ein Verstärkungsbereich mit der Drainagefolie erzielt werden kann.

Die erfindungsgemäßen Verfahren zum Herstellen einer schlauchförmigen Drainagefolie können ein Aufrollen von mindestens einem Abschnitt der schlauchförmigen Drainagefolie ausgehend von einer Schlauchöffnung an einem Schlauchende entlang einer longitudinalen Schlauchachse, insbesondere entlang einer longitudinalen Schlauchachse ausgehend von einer inneren Begrenzungsfläche in Richtung einer äußeren Begrenzungsfläche, umfassen. Die schlauchförmige Drainagefolie kann abschnittsweise oder vollständig aufgerollt werden. Es hat sich herausgestellt, dass eine Anwendung einer aufgerollten schlauchförmigen Drainagefolie gegenüber nicht aufgerollten Drainagefolien vereinfacht ist, da der Anwendende die schlauchförmige Drainagefolie mühelos über Strukturen, beispielsweise Drainageschläuche oder Sonden, abrollen kann, um diese mit der schlauchförmigen Drainagefolie zu ummanteln.

Es hat sich im Sinne einer glatten Oberfläche der Verbindungslinie auf der Außenseite der schlauchförmigen Drainagefolie als vorteilhaft erwiesen, wenn nach dem Verbinden eine der Klapp-, insbesondere Faltlinie zugewandte Seite der Verbindungslinie durch Umstülpen der schlauchförmigen Drainagefolie vollständig nach außen gedreht wird. Eine glatte Oberfläche der Verbindungslinie auf der Außenseite gewährleistet eine hohe Funktionalität der schlauchförmigen Drainagefolie im Bereich der Verbindungslinie.

Um besonders gute Drainageeffekte mit der hergestellten schlauchförmigen Drainagefolie zu erzielen, kann die in Schritt a) der erfindungsgemäßen Verfahren zum Herstellen einer schlauchförmigen Drainagefolie bereitgestellte Drainagefolie eine nach einem erfindungsgemäßen Verfahren zum Herstellen einer doppelwandigen Drainagefolie hergestellte Drainagefolie sein. Die Konstruktion der hergestellten Drainagefolie verhindert ein Kollabieren des offenen Drainageraums zwischen den beiden Lagen, unterstützt durch die Befestigungsbereiche. So ist eine Sogverteilung über die gesamte Fläche bis in die Randbereiche der schlauchförmigen Drainagefolie gewährleistet und die Drainagefolie transportiert das Exsudat effektiv ab.

### Schlauchförmige Drainagefolie

Erfindungsgemäß wird die Aufgabe der Erfindung darüber hinaus durch eine schlauchförmige Drainagefolie mit einem eine äußere Begrenzungsfläche bildenden ersten schlauchförmigen Element und einem eine der äußeren Begrenzungsfläche abgewandte innere Begrenzungsfläche bildenden und mit dem ersten schlauchförmigen Element durch mindestens einen Befestigungsbereich verbundenen zweiten schlauchförmigen Element gelöst, wobei das erste schlauchförmige Element und das zweite schlauchförmige Element jeweils mindestens eine den Durchtritt von Körperflüssigkeiten ermöglichende Öffnung umfassen und zwischen der äußeren Begrenzungsfläche und der inneren Begrenzungsfläche ein offener Drainageraum gebildet ist. Bei der erfindungsgemäßen schlauchförmigen Drainagefolie wird zwischen den beiden schlauchförmigen Elementen durch den offenen Drainageraum eine kapillare Wirkung erzielt, welche ein Abfließen bzw. Absaugen von Körperflüssigkeiten, insbesondere Exsudaten, über die gesamte schlauchförmige Struktur ermöglicht. Durch eine erfindungsgemäße schlauchförmige Drainagefolie kann ein effektives Exsudatmanagement auch von komplexeren Wunden, beispielsweise tief im Körperinneren liegenden Wunden, insbesondere sogar bei einer endoluminalen Anwendung, gewährleistet werden. Die erfindungsgemäße schlauchförmige Drainagefolie hat den Vorteil, dass sie nicht nur an der Oberfläche von Wunden angewendet werden kann, sondern auch zwischen Organstrukturen angeordnet werden kann, um dort effektiv Körperflüssigkeit, beispielsweise Exsudate, aufzunehmen.

Das erste schlauchförmige Element und/oder das zweite schlauchförmige Element können/kann eine Kunststofffolie umfassen oder sein, wobei die Kunststofffolie bevorzugt Polyethylen, Polypropylen, Polyethylenterephthalat, Polyurethan, Polytetrafluorethylen, Polyhydroxybutyrat, Polylactid und/oder Cellulose umfasst. Solche Kunststofffolien haben den Vorteil, dass sie ein Eindringen von Pathogenen, beispielsweise Bakterien, und ein Anhaften und Einwachsen von Gewebe, effektiv verhindern. Die Kunststofffolie kann insbesondere Polyethylen, bevorzugt Low-Density-Polyethylen (PE-LD) umfassen. Es hat sich herausgestellt, dass schlauchförmige Elemente bzw. Kunststofffolien mit Polyethylen, insbesondere Low-Density-Polyethylen (PE-LD), für die Herstellung und Anwendung von erfindungsgemäßen schlauchförmigen Drainagefolien besonders vorteilhaft sind. Das erste schlauchförmige Element und/oder das zweite schlauchförmige Element können/kann Polyethylen, Polypropylen, Polyethylenterephthalat, Polyurethan, Polytetrafluorethylen, Polyhydroxybutyrat, Polylactid und/oder Cellulose, bevorzugt Polyethylen, bevorzugter Low-Density-Polyethylen (PE-LD), umfassen. Im Rahmen der Erfindung ist ferner daran gedacht, dass die schlauchförmigen Elemente einseitig oder beidseitig mit einer antibakteriellen bzw. bakteriostatischen Schicht ausgestattet werden. Die antibakterielle bzw. bakteriostatische Wirkung kann bspw. durch PHMB, Silber, Chlorhexidin usw. verwirklicht werden. Im Hinblick auf die Vermeidung der Verklebungsneigung der durch das erfindungsgemäße Verfahren hergestellten Drainagefolien kann mindestens ein schlauchförmiges Element eine hydrophile oder hydrophobe Ausrüstung aufweisen. Ferner ist auch an den Auftrag von quellbaren Materialen gedacht.

Im Sinne der strukturellen Integrität der schlauchförmigen Drainagefolie hat es sich als günstig erwiesen, wenn das erste schlauchförmige Element und das zweite schlauchförmige Element an einer Vielzahl von Befestigungsbereichen, bevorzugt an einer Vielzahl von in einer Rasteranordnung ausgebildeten Befestigungsbereichen, verbunden sind. Ein Abstand zwischen benachbarten Befestigungsbereichen ist bevorzugt 2 mm oder mehr, bevorzugt 3 mm oder mehr, bevorzugter 5 mm oder mehr, um eine hervorragende kapillare Wirkung des zwischen dem ersten schlauchförmigen Element und dem zweiten schlauchförmigen Element gebildeten offenen Drainageraums zu gewährleisten. Die Vielzahl von Befestigungsbereichen kann beispielsweise in einem Rechteckraster angeordnet sein.

Es hat sich ferner hinsichtlich einer effektiven kapillaren Wirkung als günstig erwiesen, wenn die einzelnen Befestigungsbereiche in einer sich senkrecht zu einer Radialrichtung beziehungsweise Tiefenrichtung erstreckenden Schnittebene eine Fläche von 5 mm² oder weniger, insbes. 3 mm² oder weniger, besonders bevorzugt 2 mm² oder weniger, aufweisen. Die Tiefenrichtung ist insbesondere eine sich senkrecht zu den Begrenzungsflächen erstreckende Richtung. Die Radialrichtung erstreckt sich insbesondere senkrecht zur longitudinalen Schlauchachse.

Unabhängig davon, ob die Befestigung am Befestigungsbereich durch Schweißen, zum Beispiel Ultraschallverschweißen, Kleben oder andere Verbindungsarten erfolgt, kann in den Befestigungsbereichen eine die schlauchförmigen Elemente miteinander verbindenden Materialbrücke gebildet sein. Es hat sich herausgestellt, dass eine solche die Stabilität der Gesamtstruktur der schlauchförmigen Drainagefolie begünstigende Materialbrücke besonders präzise mittels Ultraschallverschweißen hergestellt werden kann. Zum Erhalt der gewünschten Kapillarwirkung im Drainageraum beträgt der Abstand zwischen den inneren Begrenzungsflächen der schlauchförmigen Elemente, welche den offenen Drainageraum begrenzen, 5 mm oder weniger, vorzugsweise 4 mm oder weniger, besonders bevorzugt 2 mm oder weniger. Dabei kann die Verteilung der Exsudate über die Gesamtfläche der schlauchförmigen Drainagefolie unter Ausnutzung der Kapillarwirkung im Drainageraum sichergestellt werden, wenn der Abstand zwischen den den Drainageraum begrenzenden inneren Begrenzungsflächen der schlauchförmigen Elemente 0,05 mm oder mehr, insbes. 0,1 mm oder mehr, besonders bevorzugt 0,3 mm oder mehr, beträgt. Die schlauchförmige Drainagefolie weist einen solchen Abstand zwischen den den Drainageraum begrenzenden inneren Begrenzungsflächen der schlauchförmigen Elemente vorzugsweise in mindestens 50 % der Fläche der Drainagefolie, bevorzugt mindestens 70 % der Fläche der Drainagefolie auf.

Die erfindungsgemäße schlauchförmige Drainagefolie kann eine Verbindungslinie, bevorzugt eine Schweißnaht, aufweisen, welche das erste schlauchförmige Element und das zweite schlauchförmige Element, und/oder das erste und/oder zweite schlauchförmige Element und ein elastisches Material, verbindet. Eine Verbindungslinie kann beispielsweise durch Schweißen, insbesondere Ultraschallverschweißen oder Laserschweißen, durch thermisches Verbinden, insbesondere mittels UV oder Heißluft, durch ein Haftmittel, insbesondere einen Klebstoff oder ein Klettverbindungsmittel, und/oder durch ein Verbindungsmittel, insbesondere eine Klammer, bereitgestellt sein. Beispielsweise kann eine Verbindungslinie dadurch entstehen, dass eine erste Kante einer Drainagefolie auf einer zweiten Kante der Drainagefolie angeordnet wird und diese, zum Beispiel durch Ultraschallverschweißen, miteinander verbunden werden, wodurch eine Verbindungslinie zwischen der ersten Kante und der zweiten Kante entsteht, beispielsweise eine Schweißnaht.

Es hat sich herausgestellt, dass es für die Funktionalität der schlauchförmigen Drainagefolie besonders vorteilhaft ist, wenn die Verbindungslinie, beispielsweise Schweißnaht, entlang einer Umfangsrichtung der schlauchförmigen Drainagefolie eine Breite von 2 mm oder weniger, bevorzugt 1 mm oder weniger, bevorzugter 0,5 mm oder weniger, aufweist. Darüber hinaus hat es sich für eine Gewährleistung der Funktionalität im gesamten Bereich der Schlauchstruktur als besonders günstig erwiesen, wenn die Verbindungslinie entlang einer Radialrichtung der schlauchförmigen Drainagefolie beziehungsweise Tiefenrichtung eine Dicke von 1 mm oder weniger, bevorzugt 750 µm oder weniger, bevorzugter 450 µm oder weniger, aufweist.

Für die Anwendung der schlauchförmigen Drainagefolie bei der Wundversorgung hat es sich als besonders vorteilhaft erwiesen, wenn die Drainagefolie ein Flächengewicht in einem Bereich von 30 g/m² bis 90 g/m², bevorzugt 40 g/m² bis 80 g/m², bevorzugter 50 g/m² bis 70 g/m², aufweist. Ein effektives Exsudatmanagement bei gleichzeitiger Sicherstellung der strukturellen Integrität der Drainagefolie kann gewährleistet werden, wenn die Drainagefolie eine Zugfestigkeit von 7 N/25,4mm oder mehr, eine Reißdehnung von 40% oder mehr, eine Porosität von 75 m³/m²/min oder mehr, und/oder eine Verbundfestigkeit von 0,5 N/25,4mm oder mehr, aufweist. Die Zugfestigkeit ist bevorzugt gemäß DIN EN ISO 527 gemessen. Die schlauchförmige Drainagefolie kann beispielsweise eine Zugfestigkeit von 7 N/25,4mm oder mehr, gemessen gemäß DIN EN ISO 527, aufweisen. Die Reißdehnung ist bevorzugt gemäß DIN EN ISO 527 gemessen. Die schlauchförmige Drainagefolie kann beispielsweise eine Reißdehnung von 40% oder mehr, gemessen gemäß DIN EN ISO 527, aufweisen. Die Verbundfestigkeit ist bevorzugt in Anlehnung an Edana NWSP 401.0.R0 gemessen. Die schlauchförmige Drainagefolie kann beispielsweise eine Verbundfestigkeit von 0,5 N/25,4mm oder mehr, gemessen in Anlehnung an Edana NWSP 401.0.R0 oder gemessen gemäß Edana NWSP 401.0.R0, aufweisen. Die Porosität ist ein Maß für das Verhältnis des Hohlraumvolumens zu dem Gesamtvolumen der Drainagefolie.

Die erfindungsgemäße schlauchförmige Drainagefolie kann an mindestens einem Schlauchende eine Schlauchöffnung aufweisen. Beispielsweise kann eine schlauchförmige Drainagefolie bereitgestellt werden, die an einem Schlauchende eine Schlauchöffnung aufweist und an dem anderen Ende der Schlauchstruktur durchgängig Material aufweist, beispielsweise mittels einer Verbindungslinie verbundene bahnförmige Elemente und/oder schlauchförmige Elemente, welche das Schlauchende bilden. Alternativ kann eine schlauchförmige Drainagefolie bereitgestellt werden, die an beiden Schlauchenden eine Schlauchöffnung aufweist. Für manche Anwendungen, beispielsweise zur Drainage bei tief im Körper liegenden Wunden, hat es sich als besonders vorteilhaft erwiesen, wenn die schlauchförmige Drainagefolie an einem Schlauchende eine Schlauchöffnung und an einem anderen Schlauchende keine Schlauchöffnung aufweist.

Erfindungsgemäß kann die schlauchförmige Drainagefolie an mindestens einem Schlauchende und/oder entlang der Verbindungslinie ein Verstärkungselement, insbesondere einen Verstärkungsstreifen oder eine Verstärkungsfolie, aufweisen. Ein Verstärkungsstreifen kann beispielsweise ein Bereich sein, in dem ein Material, beispielsweise eine doppelwandige Drainagefolie, insbesondere eine nach dem erfindungsgemäßen Verfahren zum Herstellen einer doppelwandigen Drainagefolie hergestellte Drainagefolie, doppellagig vorliegt, und/oder ein Bereich, in dem ein verstärkendes Material, beispielsweise eine Polyethylen-Folie, an dem ersten und/oder dem zweiten schlauchförmigen Element befestigt ist. Es hat sich herausgestellt, dass eine Folie, insbesondere eine Polyethylen-Folie, als Verstärkungselement einen besonders vorteilhaften Verstärkungseffekt hat. Die strukturelle Integrität der schlauchförmigen Drainagefolie wird dann besonders begünstigt, wenn die Verbindungslinie und/oder die mindestens eine Schlauchöffnung ein Verstärkungselement aufweisen/aufweist.

Die hervorragende strukturelle Integrität der erfindungsgemäßen schlauchförmigen Drainagefolie ist außerdem noch weiter gesteigert, wenn die schlauchförmige Drainagefolie an mindestens einem Schlauchende einen Verstärkungsbereich aufweist, in dem die Drainagefolie mindestens doppellagig vorliegt. Dies ist vor allem für solche Anwendungen vorteilhaft, bei denen Hilfsmittel, beispielsweise ein Absorptionskörper, Schlauch, Draht, Stent oder eine Einführhilfe, in die schlauchförmige Drainagefolie eingeschoben werden sollen. Durch den Verstärkungsbereich wird die Schlauchöffnung weiter stabilisiert, sodass beliebige Hilfsmittel in die schlauchförmige Drainagefolie eingeführt werden können, ohne dass es zu einer Gefahr des Einreißens der Drainagefolie kommt. Herstellungstechnisch hat es sich als vorteilhaft erwiesen, wenn der Verstärkungsbereich in Form eines doppellagigen Randes ausgeführt ist, der durch ein Umklappen eines Abschnitts der schlauchförmigen Drainagefolie am Schlauchende gebildet ist, wobei das Umklappen insbesondere derart durchgeführt ist, dass das die innere Begrenzungsfläche bildende zweite schlauchförmige Element im Verstärkungsbereich die äußere Begrenzungsfläche der schlauchförmigen Drainagefolie, insbesondere die äußere Begrenzungsfläche des Verstärkungsbereichs der schlauchförmigen Drainagefolie, bildet. Der umgeklappte Rand kann punktuell fixiert werden.

Erfindungsgemäß kann die äußere Begrenzungsfläche der schlauchförmigen Drainagefolie, die innere Begrenzungsfläche der schlauchförmigen Drainagefolie und/oder ein von der inneren Begrenzungsfläche der schlauchförmigen Drainagefolie umfasstes Innenlumen der schlauchförmigen Drainagefolie eine Gleithilfe und/oder eine Einführhilfe umfassen/umfasst. Durch solche Gleithilfen und/oder Einführhilfen kann der Anwendungskomfort und die Einfachheit der Anwendung weiter gesteigert werden. Beispielsweise kann eine äußere Begrenzungsfläche und/oder eine innere Begrenzungsfläche der schlauchförmigen Drainagefolie mit einer Oberfläche mit geringer Reibung, insbesondere mit einer Anti-Haft-Beschichtung und/oder einer mikrorauen Oberfläche, ausgeführt sein, um das Einführen zu erleichtern. Insbesondere kann die äußere Begrenzungsfläche und/oder die innere Begrenzungsfläche der schlauchförmigen Drainagefolie Silikon, Polytetrafluorethylen (PTFE) und/oder Feuchtigkeit umfassen oder damit beschichtet sein, um das Einführen zu erleichtern. Gleithilfen, welche die Gleitfähigkeit deutlich steigern, sind beispielsweise ein wasserbasiertes Gleitmittel, ein glycerolbasiertes Gleitmittel, ein polymeres Gel, insbesondere ein antiallergenes polymeres Gel, ein endoskopisches Gleitmittel, insbesondere ein endoskopisches Gleitgel, und/oder eine wässrige Lösung, insbesondere eine Natriumchlorid-Lösung. Es ist besonders vorteilhaft, wenn sich die als Gleithilfen verwendeten Substanzen rückstandslos, insbesondere in einer wässrigen Lösung, auflösen können, damit verhindert wird, dass Substanzreste auf dem Produkt zurückbleiben. Als Einführhilfen eignen sich besonders Einführhilfen, die ein Textil und/oder einen Kunststoff umfassen. Die Einführhilfen können eine Oberfläche mit geringer Reibung, insbesondere eine Anti-Haft-Beschichtung und/oder eine mikroraue Oberfläche, umfassen. Beispielsweise kann die Einführhilfe ein Textil und/oder einen Kunststoff umfassen, wobei das Textil bzw. der Kunststoff Silikon, Polytetrafluorethylen (PTFE) und/oder Feuchtigkeit umfasst oder damit beschichtet. Die Einführhilfe kann ein in dem Innenlumen der schlauchförmigen Drainagefolie angeordnete schlauchförmige Einführhilfe sein, beispielsweise eine schlauchförmige Einführhilfe, welche eine Schlauchöffnung oder zwei Schlauchöffnungen aufweist. Insbesondere kann die Einführhilfe an einem Schlauchende eine Schlauchöffnung aufweisen und an dem anderen Schlauchende ein geschlossenes Schlauchende aufweisen. Eine schlauchförmige Einführhilfe kann mit einer Sollbruchstelle an einem geschlossenen Schlauchende der schlauchförmigen Einführhilfe ausgeführt sein. Die schlauchförmige Drainagefolie kann einlumig oder mehrlumig bereitgestellt sein.

Damit die schlauchförmige Drainagefolie für eine Vielzahl von Anwendungen, beispielsweise einer endoluminalen Anwendung, einer Anwendung in der Gynäkologie oder Urologie, und einer Anwendung in der Thorax-, Viszeral- und/oder Hüftchirurgie, besonders vorteilhaft ist, kann die schlauchförmige Drainagefolie eine Länge in einem Bereich von 0,5 cm bis 40 cm, bevorzugt 2 cm bis 30 cm, bevorzugter 5 cm bis 20 cm, aufweisen. Es können jedoch auch längere oder kürzere schlauchförmige Drainagefolien bereitgestellt werden. Für intraluminale Anwendungen im Gastrointestinaltrakt sowie für Fistelgänge sind beispielsweise schlauchförmige Drainagefolien mit einer Länge von 10 cm oder länger, bzw. 20cm oder länger geeignet. Bei einer Anwendung in verschiedenen Wundhöhlen kann die Länge an die Wundhöhle angepasst werden, beispielsweise können für Wundhöhlen schlauchförmige Drainagefolien mit einer Länge von 10cm oder weniger, beziehungsweise für kleinere Wundhöhlen mit Längen von 5 cm oder weniger, zum Beispiel 2 cm oder weniger, bereitgestellt sein.

Die schlauchförmige Drainagefolie ist darüber hinaus für eine Vielzahl von Anwendungen im Sinne eines effektiven Wundmanagements besonders geeignet, wenn die schlauchförmige Drainagefolie einen Außendurchmesser in einem Bereich von 1 mm bis 60 mm, bevorzugt 2 mm bis 50 mm, bevorzugter 4 mm bis 30 mm, aufweist. Es können jedoch auch schlauchförmige Drainagefolien mit größerem oder kleinerem Außendurchmesser bereitgestellt werden. Für Anwendungen bei schmalen Wunden und/oder in tiefen Körperregionen, beispielsweise am Pankreas, Anwendungen in der Gynäkologie oder Urologie, und bei endoluminalen Anwendungen ist eine schlauchförmige Drainagefolie mit geringem Außendurchmesser besonders geeignet, beispielsweise schlauchförmige Drainagefolien mit einem Außendurchmesser von 5 mm oder weniger, insbesondere 4 mm oder weniger. Die erfindungsgemäße schlauchförmige Drainagefolie hat den Vorteil, dass selbst bei einem geringen Außendurchmesser der Drainagefolie eine gleichmäßige Drainageleistung gewährleistet ist und ein Verstopfen des Drainageabflusses durch Gewebe verhindert wird. Die schlauchförmige Drainagefolie Folie ermöglicht somit ein aktives Ableiten von Flüssigkeiten sogar bei sehr geringem Außendurchmesser. Bei invasiver Chirurgie, z.B. an der Hüfte, insbesondere bei großen Wundregionen, können auch schlauchförmige Drainagefolien mit größerem Außendurchmesser verwendet werden, beispielsweise mit einem Außendurchmesser von 6 mm oder mehr. Entsprechend der Zugänglichkeit und Größe der Wundregion kann eine schlauchförmige Drainagefolie mit geeigneter Länge und geeignetem Außendurchmesser bereitgestellt werden.

### Wundversorgungskit

Die Aufgabe der Erfindung wird darüber hinaus durch ein erfindungsgemäßes Wundversorgungskit gelöst, welches eine nach dem erfindungsgemäßen Verfahren zum Herstellen einer schlauchförmige Drainagefolie hergestellte schlauchförmige Drainagefolie oder die erfindungsgemäße schlauchförmige Drainagefolie, und mindestens einen weiteren Bestandteil, beispielsweise ausgewählt aus einem Absorptionskörper, einem Schlauch, einem Draht, einem Stent und einer Einführhilfe, umfasst. Im Sinne einer besonders einfachen Handhabung des Wundversorgungskits hat es sich als vorteilhaft erwiesen, wenn mindestens ein Abschnitt des mindestens einen weiteren Bestandteils in einem von einer inneren Begrenzungsfläche umfassten Innenlumen der schlauchförmigen Drainagefolie angeordnet ist. Für eine besonders effiziente Unterdrucktherapie kann das Wundversorgungskit beispielsweise die schlauchförmige Drainagefolie und einen Drainageschlauch umfassen, wobei ein Abschnitt des Drainageschlauches in einem Innenlumen der schlauchförmigen Drainagefolie angeordnet ist. Der Drainageschlauch kann an eine Unterdruckquelle angeschlossen sein oder werden. Der mindestens eine weitere Bestandteil kann abschnittsweise oder vollständig in der schlauchförmigen Drainagefolie angeordnet sein.

Bei einer besonders bevorzugten Ausführungsform der Erfindung ist der Absorptionskörper ein Schwamm, Schaum, insbesondere Polyurethan- oder Polyvinylalkohol-Schaum, oder Gaze. Es hat sich herausgestellt, dass ein Wundversorgungskit mit einem solchen Absorptionskörper eine effiziente Absaugung von Flüssigkeiten aus Körperregionen, insbesondere Wundregionen, ermöglicht. Das Anhaften von Gewebe an dem Absorptionskörper wird verhindert, wenn der Absorptionskörper in dem von einer inneren Begrenzungsfläche umfassten Innenlumen der schlauchförmigen Drainagefolie angeordnet ist. Flüssigkeiten können so effizient und atraumatisch aus Wundregionen abgesaugt werden.

Im Sinne einer besonders vorteilhaften Absaugungseffizienz hat es sich als günstig erwiesen, wenn ein von einem Wundversorgungskit umfasster Schlauch ein Drainageschlauch ist. Der Drainageschlauch kann einlumig oder mehrlumig sein. Ein Wundversorgungskit mit einem Drainageschlauch ermöglicht ein stetiges Absaugen von Flüssigkeiten aus der Wundregion, und ermöglicht ferner ein Absaugen von großen Flüssigkeitsvolumina. Dadurch, dass die schlauchförmige Drainagefolie in jeglicher Dimensionierung, insbesondere mit jeglichem Außendurchmesser, jeglicher Länge und jeglicher Form, bereitgestellt werden kann, kann das Wundversorgungskit sogar mit Schläuchen bereitgestellt werden und/oder mit Schläuchen verwendet werden, welche eine komplexe Geometrie, beispielsweise eine mehrlumige Geometrie, aufweisen.

Das erfindungsgemäße Wundversorgungskit kann eine Länge in einem Bereich von 0,5 cm bis 250 cm, bevorzugt 10 cm bis 200 cm, aufweisen. Die Länge des Wundversorgungskits kann für die jeweilige Anwendung optimiert sein, beispielsweise kann ein Wundversorgungskit mit einer Länge von mindestens 10 cm oder mindestens 20 cm für eine intraluminale Anwendungen im Gastrointestinaltrakt oder für eine Anwendung in Fistelgängen bereitgestellt werden. Für eine Anwendung in Wundhöhlen kann die Länge an die jeweilige Wundhöhle angepasst sein, beispielsweise kann das Wundversorgungskit, je nach Größe beziehungsweise Tiefe der Wundhöhle, eine Länge von weniger als 10 cm, weniger als 5 cm, oder weniger als 2 cm aufweisen.

### Medizinische Verwendung und Verfahren zum Versorgen einer Wunde

Die Erfindung bezieht sich darüber hinaus auf die erfindungsgemäße schlauchförmige Drainagefolie zur Verwendung bei einer Wundversorgung und das erfindungsgemäße Wundversorgungskit zur Verwendung bei einer Wundversorgung, beispielsweise zur Drainage einer Wunde und/oder zur Verwendung bei einem erfindungsgemäßen Verfahren zum Versorgen einer Wunde. Die schlauchförmige Drainagefolie bzw. das Wundversorgungskit werden bevorzugt zur Drainage, beispielsweise während chirurgischen Operationen und/oder postoperativ eingesetzt. Bevorzugt wird die erfindungsgemäße schlauchförmige Drainagefolie beziehungsweise das erfindungsgemäße Wundversorgungskit zur Drainage von Körperflüssigkeiten aus Wundregionen, beispielsweise während Operationen, verwendet. Die erfindungsgemäße schlauchförmige Drainagefolie und das erfindungsgemäße Wundversorgungskit ermöglichen ein einfaches und sicheres Absaugen von Flüssigkeiten aus einer Wunde. Die erfindungsgemäße schlauchförmige Drainagefolie sowie das erfindungsgemäße Wundversorgungskit können beispielsweise endoluminal z.B. im Gastrointestinaltrakt, in der Gynäkologie und Urologie, bei chirurgischen Operationen, beispielsweise bei der Thorax-, Viszeral- und Hüftchirurgie, und bei der Unterdrucktherapie angewendet werden. Die schlauchförmige Drainagefolie bzw. das Wundversorgungskit können zur Verwendung bei einer Drainage, insbesondere im Rahmen einer Operation, eingesetzt werden. So sammelt sich bei einem chirurgischen Eingriff meist Blut, Wundsekrete und/oder Gewebsflüssigkeit an, deren Ansammlung in der Wundhöhle durch das Anlegen der Drainage verhindert werden kann. Auf diese Weise kann der Heilungsprozess deutlich erleichtert werden.

Die erfindungsgemäße schlauchförmige Drainagefolie bzw. das Wundversorgungskit sind mit besonderem Vorteil in Kombination mit der Unterdrucktherapie zur Wundversorgung einsetzbar. Dabei wird die erfindungsgemäße schlauchförmige Drainagefolie bzw. das Wundversorgungskit als direktes Interface zwischen der Wunde, insbesondere dem Wundgrund, und einem Wundfüller bzw. einem Bereich des Exsudatabtransports, beispielsweise einem Drainageschlauch, eingesetzt. Mit besonderem Vorteil ist die erfindungsgemäße schlauchförmige Drainagefolie bzw. das Wundversorgungskit für den Einsatz bei Wunden an den inneren Organen einsetzbar. Dabei wird die schlauchförmige Drainagefolie an der Wunde angelegt, wobei die schlauchförmige Drainagefolie eine Drainagefunktion und eine sehr glatte Oberfläche bietet, um ein Anhaften bzw. eine Friktion von Gewebe und Folie zu verhindern. Die erfindungsgemäße schlauchförmige Drainagefolie bzw. das Wundversorgungskit kann anatomisch vorgeformt, aber auch auf spezielle Gegebenheiten zuschneidbar ausgeführt sein. In die erfindungsgemäße schlauchförmige Drainagefolie bzw. das Wundversorgungskit kann bei Einsatz in Kombination mit der Unterdrucktherapie ein Füllmedium wie Gaze oder Schaum gefüllt werden und/oder ein Drainageschlauch eingelegt werden. Der zur Ableitung der Exsudate bzw. zur Wunddrainage eingesetzte Schlauch kann aus dem Körperinneren, beispielsweise dem Bauchraum, herausgeführt und an eine Unterdruckquelle angeschlossen werden, die durch eine Pumpe bzw. im Krankenhaus durch eine zentrale Hausversorgung sichergestellt werden kann. Mit der Unterdruckquelle kann dann das Exsudat aus der Wunde mittels der erfindungsgemäßen schlauchförmigen Drainagefolie bzw. dem Wundversorgungskit abtransportiert werden, wodurch ein gutes Exsudatmanagement erreicht wird.

Bei einer medizinischen Verwendung der erfindungsgemäßen schlauchförmigen Drainagefolie bzw. dem Wundversorgungskit ergeben sich die folgenden Vorteile:
- Die den Durchtritt von Körperflüssigkeiten ermöglichenden Öffnungen unterstützen den Abtransport von Exsudat und verhindern das Zurückfließen des Exsudats in den Wundraum (Kapillarwirkung).
- Die schlauchförmige Drainagefolie verhindert ein Anhaften von Gewebe bzw. Zellen am Wundgrund bzw. bei den umliegenden Organen.
- Die schlauchförmige Drainagefolie bzw. das Wundversorgungskit ist ohne Beeinträchtigung der Drainagefunktion auf jede Größe bzw. Struktur der Wundregion zuschneidbar und ermöglicht so auch eine Versorgung komplexer Wunden.

Nachstehend wird die Erfindung unter Bezugnahme auf die Zeichnung, auf die hinsichtlich aller erfindungswesentlichen und in der Beschreibung nicht näher herausgestellten Einzelheiten ausdrücklich verwiesen wird, erläutert. In der Zeichnung zeigt:
- **Fig. 1**: eine schematische Darstellung einer Ultraschallverschweißvorrichtung
- **Fig. 2**: eine schematische Darstellung einer Stanzvorrichtung
- **Fig. 3**: eine schematische Darstellung einer Vorrichtung zum Umstülpen der schlauchförmigen Drainagefolie
- **Fig. 4**: eine schematische Darstellung einer Falttechnik bzw. Wickeltechnik zum Umklappen, insbesondere Falten der Drainagefolie um eine Klapp-, insbesondere Faltlinie, oder zum Wickeln der Drainagefolie um eine Wickelach- se derart, dass die Drainagefolie einen zu einer Verbindungslinie verbindbaren Überlappungsbereich aufweist
- **Fig. 5**: eine schematische Darstellung einer selbsthaftenden Drainagefolie, welche durch Wickeln bzw. Falten als schlauchförmige Drainagefolie eingesetzt werden kann
- **Fig. 6**: eine schematische Darstellung einer selbsthaftenden Drainagefolie, welche durch Wickeln bzw. Falten, beispielsweise um einen Drainageschlauch, als schlauchförmige Drainagefolie eingesetzt werden kann
- **Fig. 7**: eine schematische Darstellung einer Verbindungslinie, insbesondere einer Schweißnaht
- **Fig. 8**: eine schematische Darstellung eines Anbringens eines Verstärkungselements an der Drainagefolie
- **Fig. 9**: eine schematische Darstellung einer Einführhilfe
- **Fig. 10**: eine schematische Darstellung eines Aufrollens der schlauchförmigen Drainagefolie sowie einer aufgerollten schlauchförmigen Drainagefolie
- **Fig. 11**: eine schematische Darstellung einer schlauchförmigen Drainagefolie mit elastischem Material
- **Fig. 12**: eine schematische Darstellung einer schlauchförmigen Drainagefolie mit durch Umklappen verstärktem Rand
- **Fig. 13**: schematische Darstellungen von schlauchförmigen Drainagefolien
- **Fig. 14**: eine schematische Darstellung einer schlauchförmigen Drainagefolie mit Schlauch
- **Fig. 15**: eine schematische Darstellung einer schlauchförmigen Drainagefolie mit Schlauch und Absorptionskörper
- **Fig. 16**: eine schematische Darstellung einer gewickelten schlauchförmigen Drainagefolie mit Schlauch.

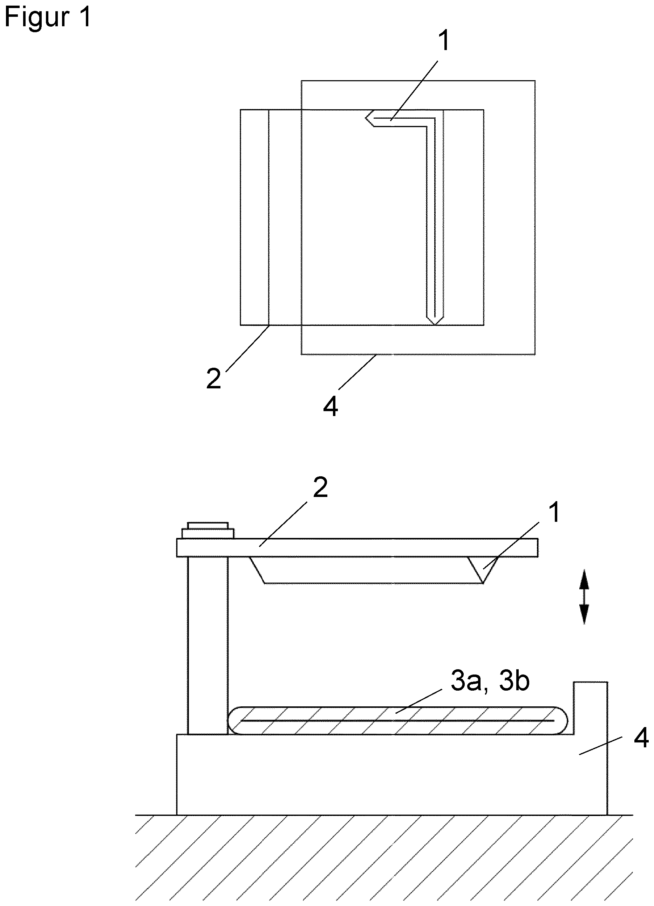

Die in Fig. 1 dargestellte Ultraschallverschweißvorrichtung kann zur Herstellung der erfindungsgemäßen schlauchförmigen Drainagefolie 3a, eingesetzt werden. Beispielsweise kann die Ultraschallverschweißvorrichtung eingesetzt werden, um eine schlauchförmige Drainagefolie 3a durch gleichzeitiges Stanzen und Schweißen herzustellen; hierbei wird durch das Ultraschallverschweißen das überlappende Material im Überlappungsbereich zu einer Verbindungslinie 11 verbunden und gleichzeitig ein Randstreifen aus restlichem Material entfernt. Die doppelwandige Drainagefolie 3b wird umgeklappt in das Fundament 4 eingelegt und fixiert. Im über dem Fundament 4 positionierten Bügel 2 befindet sich die Ultraschall-Schweißform 1, beispielsweise eine L-förmige Ultraschall-Schweißform 1. Ein Generator induziert die benötigte Spannung in die Schweißform 1. Die Schweißform 1 fährt nach unten und schweißt die Kontur der schlauchförmigen Drainagefolie 3a aus. Nachdem der Schweißvorgang beendet ist, fährt der Bügel 2 wieder in die Ausgangsposition. Die schlauchförmige Drainagefolie 3a kann dann entnommen werden. Die linke Darstellung zeigt die Aufsicht auf die Ultraschallverschweißvorrichtung und die rechte Darstellung zeigt eine Seitenansicht.

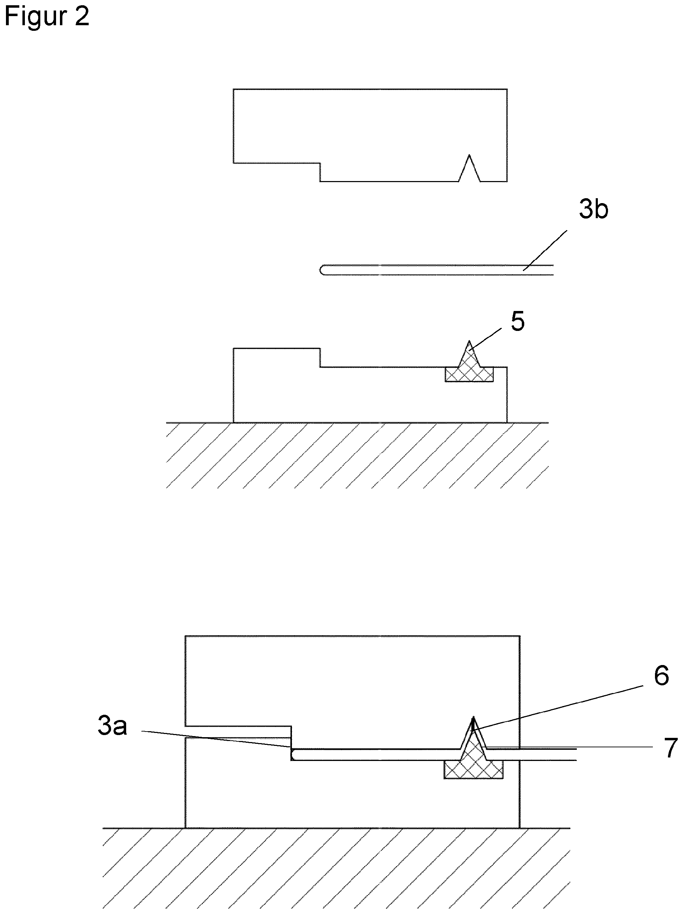

Die in Fig. 2 dargestellte Stanzvorrichtung kann zum Herstellen der erfindungsgemäßen Drainagefolie 3 eingesetzt werden. Eine bereitgestellte doppelwandige Drainagefolie 3b wird an einer Seite umgeklappt in die untere Formhälfte des Stanzwerkzeuges eingelegt. In der unteren Formhälfte des Stanzwerkzeuges befindet sich ein Schneidwerkzeug mit integrierter Heizung. Durch das Herunterfahren der oberen Formhälfte wird der Stanzvorgang gestartet. Durch das warme und scharfe Schneidelement 5 wird im selben Vorgang die Drainagefolie 3b an der Schneidekante verschweißt und getrennt, wodurch eine schlauchförmige Drainagefolie 3a mit Schweißnaht 6 erhalten wird. Der Folienrest 7 wird durch das Stanzen entfernt. Anschließend wird die Form durch Hinauffahren der oberen Formhälfte geöffnet und die schlauchförmige Drainagefolie 3a kann entnommen werden.

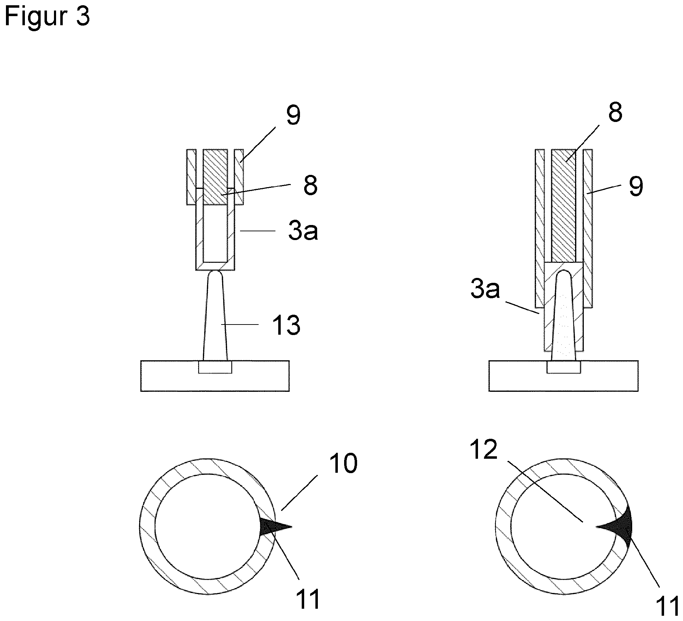

Die in Fig. 3 dargestellte Vorrichtung kann zum Umstülpen der schlauchförmigen Drainagefolie 3a eingesetzt werden. Zuerst wird die schlauchförmige Drainagefolie 3a nach einem der angeführten Verfahren, beispielsweise durch Verschweißen des Überlappungsbereichs zu einer Verbindungslinie 11, hergestellt. Es hat sich für die Anwendung in der Wundregion, insbesondere zum Verhindern eines Anhaftens von Gewebe, als besonders vorteilhaft erwiesen, wenn an der Außenseite der schlauchförmigen Drainagefolie 3a die Verbindungslinie 11 eine glatte Oberfläche aufweist. Um eine glatte Oberfläche an der Schweißnaht 6 zu erzeugen, kann die schlauchförmige Drainagefolie 3a von innen nach außen derart umgedreht werden, dass die zuvor der Klapp-, insbesondere Faltlinie abgewandte Seite 10 der Verbindungslinie 11 nach dem Umstülpen auf der Klapp-, insbesondere Faltlinie zugewandten Seite 12 der Verbindungslinie 11 liegt. Die schlauchförmige Drainagefolie 3a kann zum Umdrehen bzw. Umstülpen über den oberen beweglichen Stößel 8 der dargestellten Vorrichtung gezogen werden. Eine Hülse 9 fährt zusätzlich über den beweglichen Stößel 8 und dreht mit Hilfe des starren Gegenstößels 13 die schlauchförmige Drainagefolie 3a um.

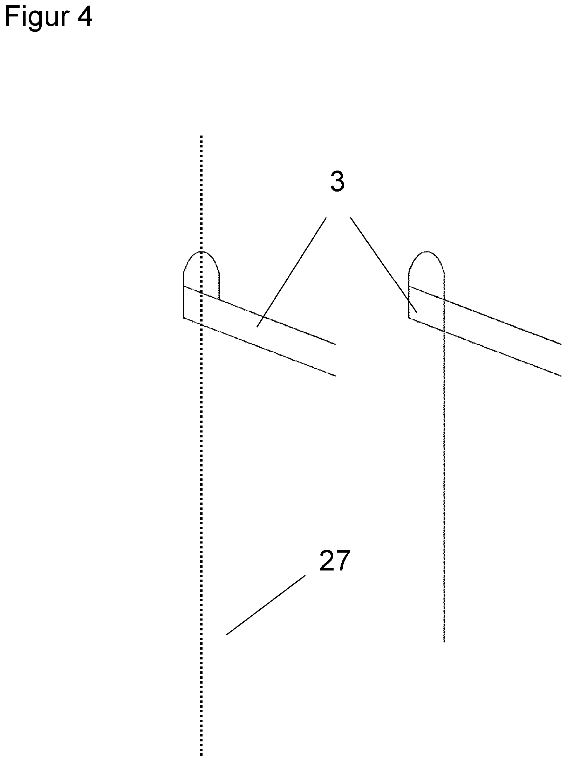

Die in Fig. 4 dargestellte Umklapp- bzw. Wickeltechnik kann dazu verwendet werden, eine Drainagefolie 3 flächig um eine Wickelachse 27, beispielsweise über die Spitze eines Schlauchs 16, zu legen oder über einen Schlauch 16 zu falten. Beispielsweise kann die Drainagefolie 3 um eine Wickelachse 27 derart gewickelt werden, dass die Drainagefolie 3 einen durch das Wickeln um die Wickelachse 27 gebildeten, zu einer Verbindungslinie 11 verbindbaren Überlappungsbereich aufweist, in dem die Drainagefolie 3 mindestens doppellagig vorliegt. Der Überlappungsbereich kann dann zum Erhalt einer schlauchförmigen Drainagefolie 3a verbunden bzw. fixiert werden, beispielsweise durch ein Haftmittel 14 oder ein Verbindungsmittel, wie eine Klammer, Naht oder eine ringförmige Applikationshilfe.

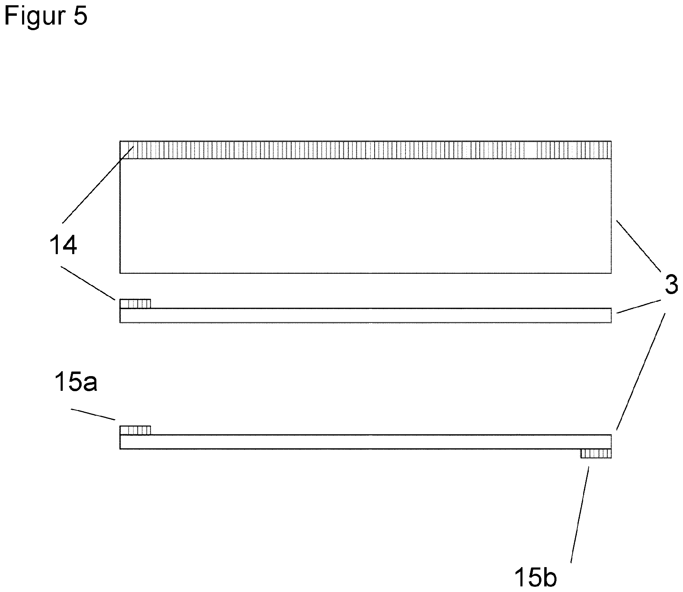

Die in Fig. 5 dargestellte selbsthaftende Drainagefolie 3 kann an beliebigen Strukturen, befestigt werden, beispielsweise an Körperteilen, Operationsvorrichtungen, Absorptionskörpern, Schläuchen, Drähten, Stents, Einführhilfen 22, und/oder Vorrichtungen zur Unterdrucktherapie. Ein an der Drainagefolie 3 angebrachtes Haftmittel 14, insbesondere ein Klebstoff, ein adhäsives Material und/oder ein Klettverbindungsmittel 15, wie ein oder mehrere Widerhaken, kann dazu verwendet werden, dass die Drainagefolie 3 um andere Strukturen gewickelt bzw. gefaltet und dann insbesondere schlauchförmig an der Struktur oder um die Struktur herum befestigt werden kann. Eine solche Drainagefolie 3 kann als selbsthaftende schlauchförmige Drainagefolie 3a eingesetzt werden und hat den Vorteil, dass sie in situ an die strukturellen Anforderungen, beispielsweise an die Wundhöhlengröße, angepasst werden kann. Bei dem Wickeln bzw. Falten der Drainagefolie 3 um die entsprechende Struktur, beispielsweise um einen Drainageschlauch, kann der klebende bzw. haftende Abschnitt der Drainagefolie 3 an einem anderen Abschnitt der Drainagefolie 3 befestigt werden. Wenn die so an einer Struktur fixierte Drainagefolie 3 besonders hohen mechanischen Belastungen standhalten muss, kann die Drainagefolie 3 bei Bedarf mittels einer Naht zusätzlich fixiert werden. Erfindungsgemäß kann die Drainagefolie 3 mit einem oder mehreren haftenden Abschnitten bereitgestellt werden, insbesondere kann das Haftmittel 14 an einem Abschnitt angebracht werden, oder das Haftmittel 14, beispielsweise die zwei komplementären Bestandteile eines Klettverschlusses 15a, 15b, kann an zwei oder mehr Abschnitten angebracht werden, welche dadurch aneinander anheftbar sind.

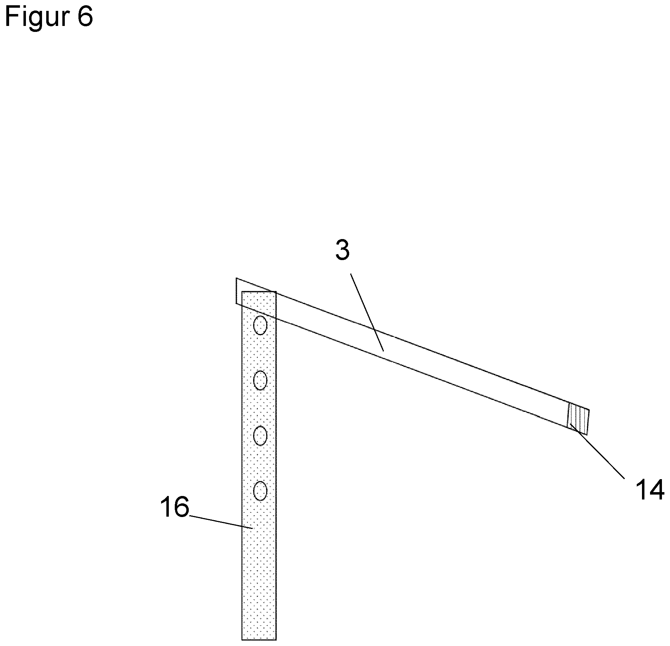

Fig. 6 zeigt eine selbsthaftende Drainagefolie 3 mit einem Haftmittel 14, welche um einen Schlauch 16 bzw. eine Sonde gewickelt werden kann und so als schlauchförmige Drainagefolie 3a einsetzbar ist.

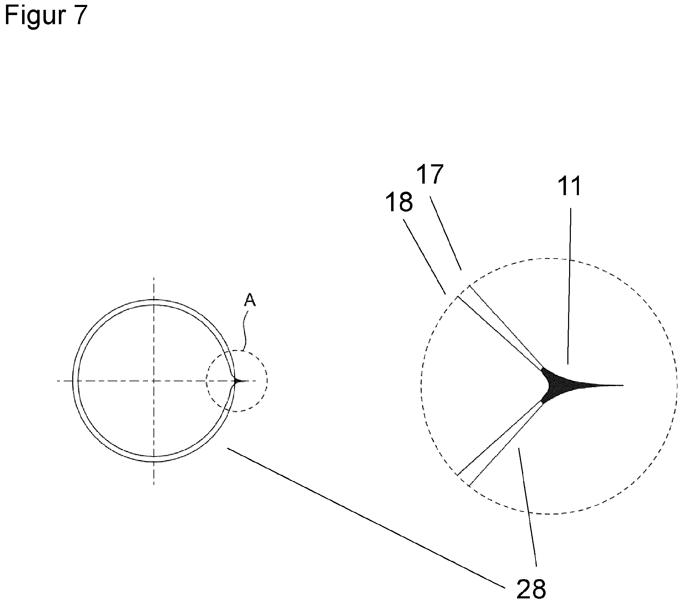

Fig. 7 zeigt eine schematische Darstellung einer Verbindungslinie 11, insbesondere Schweißnaht 6, zwischen einem ersten Element 17 und einem zweiten Element 18, beispielsweise einem ersten und zweiten bahnförmigen Element bzw. einem ersten und zweiten schlauchförmigen Element. Das Detail A zeigt eine Verjüngung der Foliendicke durch das Verbinden, insbesondere durch einen Schweißvorgang. Die Folie wird im Bereich der Verbindungslinie 11, insbesondere Schweißnaht 6, zusammengedrückt. Durch das Komprimieren der doppelwandigen Drainagefolie 3b im Bereich der Verbindungslinie 11 und den Schneidradwinkel entsteht ein Geometriebedingte Winkel α, zwischen dem Grat der Verbindungslinie 11 und der äußere Begrenzungsfläche 28 der schlauchförmigen Drainagefolie 3a.

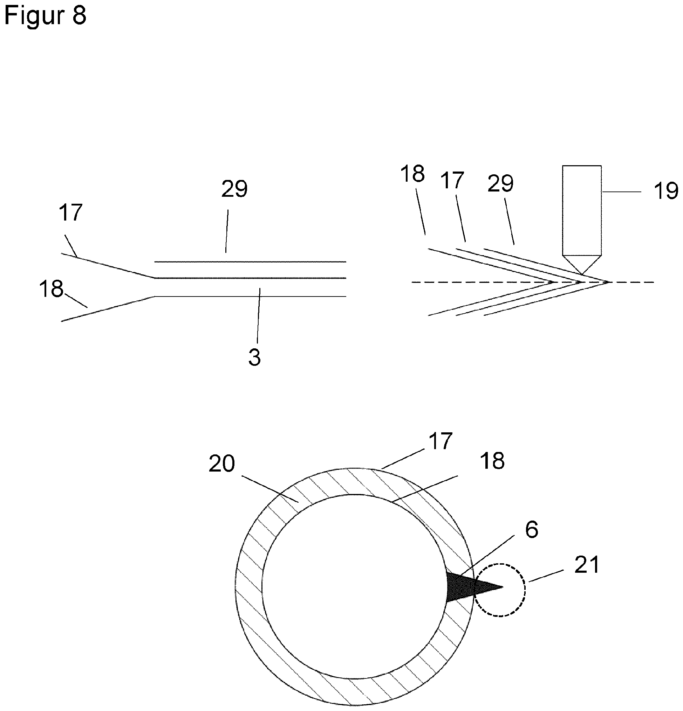

Eine Verbindungslinie 11, beispielsweise eine Schweißnaht 6, einer perforierten Drainagefolie 3 kann im Sinne der Folienstabilität eine kritische Stelle des Produkts darstellen. Um die erfindungsgemäßen schlauchförmigen Drainagefolie 3a mit offenem Drainageraum 20 mit besonders hervorragenden Festigkeitseigenschaften auszustatten, insbesondere eine Festigkeit einer Verbindungslinie 11 wie einer Schweißnaht 6 zu verbessern, kann diese mit zusätzlichem Material verstärkt werden. Fig. 8 zeigt eine schematische Darstellung eines solchen Anbringens eines Verstärkungselements 29 an der das erste Element 17 und das zweite Element 18 umfassenden Drainagefolie 3, beispielsweise durch ein Verschweißen mit einem Schneidrad 19. Als zusätzliches Material bzw. als Verstärkungselement 29 kann eine insbesondere nicht-perforierte Polyethylen-Folie verwendet werden. Um die zusätzliche Folienschicht, beispielsweise zusätzliche insbesondere nicht-perforierte Polyethylen-Folie, mit der Drainagefolie 3 besonders effektiv verschweißen zu können, kann die Folienschicht aus Polyethylen sein. Durch die zusätzlich verschweißte insbesondere nicht-perforierte Polyethylen-Folie fließt im Bereich der Verbindungslinie 11, beispielsweise beim Verschweißen, mehr Material in die Löcher der Drainagefolie 3 und stabilisiert diese, wodurch eine hervorragende Festigkeit erreicht wird. Ein solches Verstärkungselement 29, beispielsweise eine Polyethylen-Folie, erhöht deutlich die Zugbelastung der Schweißnaht 6. Um das Verstärkungselement 29 anzubringen, kann das Verstärkungselement, beispielsweise eine zusätzliche Polyethylen-Folie, über die Drainagefolie 3 gelegt und anschließend umgeklappt werden. Während dem Verbinden, beispielsweise durch Ultraschallverschweißen, verbindet sich das Verstärkungselement 29 mit der Drainagefolie 3 im Überlappungsbereich, wodurch eine Verbindungslinie 11, insbesondere eine stabile Schweißnaht 6 entsteht. Das restliche Material 21 des Verstärkungselements 29, beispielsweise eine restliche Polyethylen-Folie, kann dann im Bereich der Verbindungslinie 11, insbesondere im Bereich der Schweißnaht 6, entfernt werden, z.B. händisch oder maschinell. Das Verstärkungselement 29, beispielsweise eine Polyethylen-Folie, kann so bereitgestellt werden, dass es nur in der Verbindungslinie 11, insbesondere einer Schweißnaht 6, vorhanden ist.

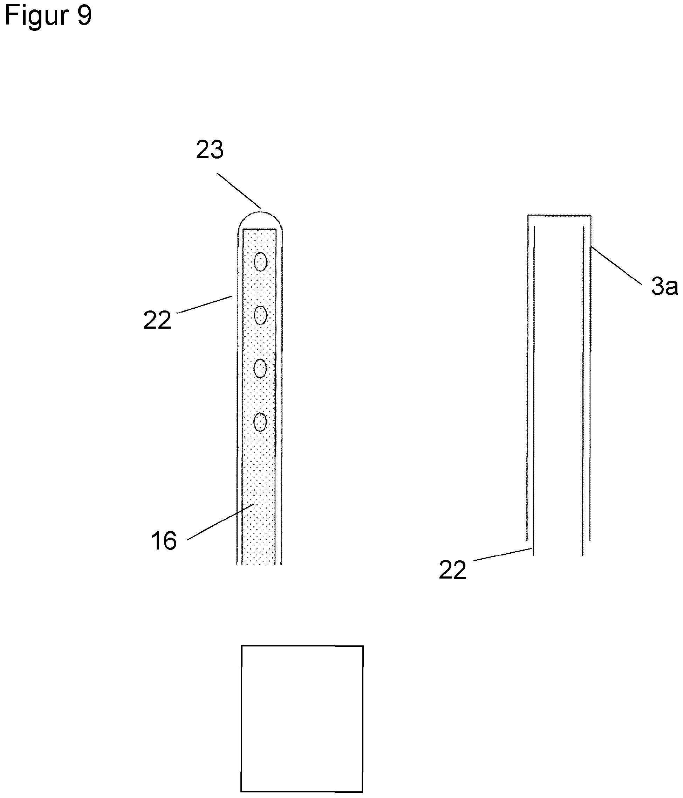

Die in Fig. 9 gezeigte schlauchförmige Drainagefolie 3a mit Einführhilfe 22 vereinfacht die Applikation der Drainagefolie 3. Als Einführhilfe 22 können diverse Materialien wie Textilien oder Kunststoffe verwendet werden, welche eine geringe Reibung gegenüber Kunststoffen aufweisen, z.B. mikroraue Textilien. Die Einführhilfe 22 kann sowohl auf den Schlauch 16 aufgebracht werden (Fig. 9 links) oder auch direkt in der Drainagefolie 3a integriert werden bzw. in einem Innenlumen der schlauchförmigen Drainagefolie 3a umfasst sein (Fig. 9 rechts). Beispielsweise kann die Einführhilfe 22 über einen Schlauch, beispielsweise Drainageschlauch oder eine Sonde, gestülpt werden (Fig. 9 links). Die schlauchförmige Drainagefolie 3a kann, beispielsweise wenn mit der schlauchförmigen Drainagefolie 3a ein Schlauch 16 ummantelt werden soll, ein geschlossenes Schlauchende aufweisen. Es hat sich für manche Anwendungen als vorteilhaft herausgestellt, wenn ein solches geschlossenes Schlauchende mit einer Sollbruchstelle 23 versehen ist. Durch eine Sollbruchstelle 23 kann durch Krafteinsatz die Einführhilfe 22 nach der Applikation entfernt werden. Die Einführhilfe 22 kann auch schlauchförmig mit zwei offenen Schlauchenden ausgeführt sein (Fig. 9 rechts). Herstellungstechnisch hat es sich als besonders vorteilhaft herausgestellt, wenn die Einführhilfe 22 bereits bei der Herstellung der schlauchförmigen Drainagefolie 3a in die schlauchförmige Drainagefolie 3a eingearbeitet wird. Für eine einfache Handhabung der Einführhilfe 22 hat es sich als günstig erwiesen, wenn die Einführhilfe 22 länger als die schlauchförmige Drainagefolie 3a ist, damit diese an einem Schlauchende der schlauchförmigen Drainagefolie 3a herausragt und greifbar ist. Nach Einschieben der schlauchförmigen Drainagefolie 3a bzw. eines von einer schlauchförmigen Drainagefolie 3a ummantelten Schlauches in die Wundregion kann die Einführhilfe 22 durch einfaches Herausziehen entfernt werden.

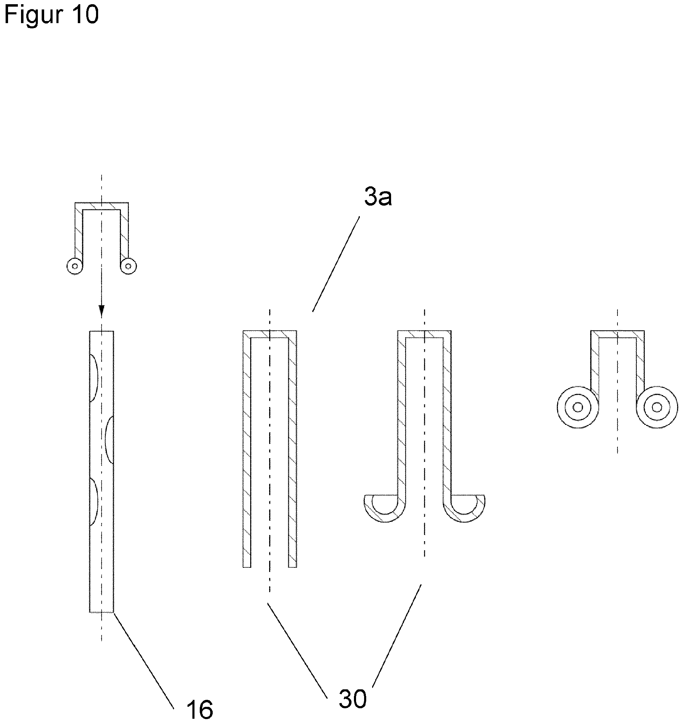

In Fig. 10 ist ein Aufrollen der schlauchförmigen Drainagefolie 3a dargestellt, um eine aufgerollte schlauchförmige Drainagefolie 3a zu erhalten. Abschnittsweise oder vollständig aufgerollte schlauchförmige Drainagefolien 3a haben den Vorteil, dass sie effizient, insbesondere durch Materialeinsparung umweltfreundlich, verpackt werden können. Darüber hinaus haben Sie den Vorteil, dass der Anwendende die schlauchförmige Drainagefolie 3a mühelos über Strukturen, beispielsweise Schläuche 16, insbesondere Drainageschläuche oder Sonden, abrollen kann, um diese mit der schlauchförmigen Drainagefolie 3a zu ummanteln. Zum Aufrollen der schlauchförmigen Drainagefolie 3a kann ein Abschnitt der schlauchförmigen Drainagefolie 3a oder die vollständige schlauchförmige Drainagefolie 3a ausgehend von einer Schlauchöffnung an einem Schlauchende entlang einer longitudinalen Schlauchachse 30 aufgerollt werden. Insbesondere kann die schlauchförmige Drainagefolie 3a entlang der longitudinalen Schlauchachse 30 ausgehend von einer inneren Begrenzungsfläche in Richtung einer äußere Begrenzungsfläche 28 aufgerollt werden, um einen Abschnitt oder die gesamte schlauchförmigen Drainagefolie 3a aufzurollen.

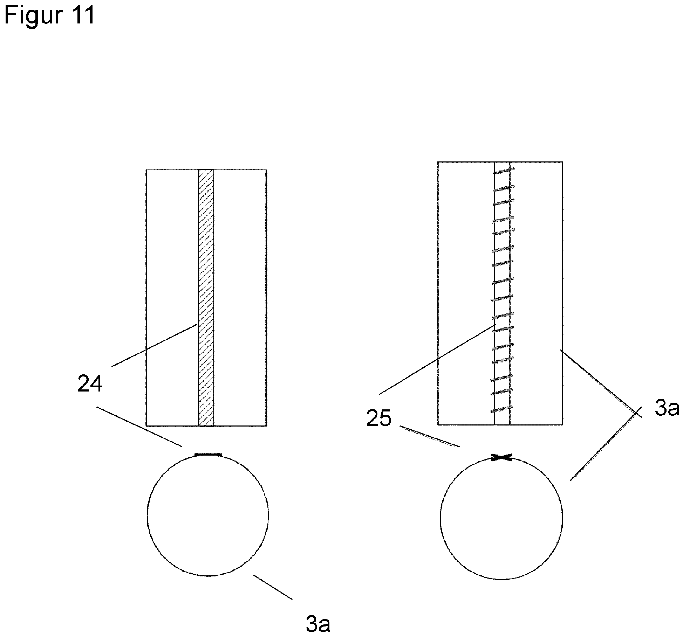

In Fig. 11 ist eine schlauchförmige Drainagefolie 3a mit elastischem Material gezeigt. Die schlauchförmige Drainagefolie 3a kann beispielsweise eine elastische Folie 24 oder eine elastische Naht 25, insbesondere eine Naht aus einem elastischen Material, aufweisen. Durch das Integrieren eines elastischen Materials in eine schlauchförmige Drainagefolie 3a kann die Elastizität der schlauchförmigen Drainagefolie 3a, insbesondere wenn die zur Bildung der schlauchförmigen Drainagefolie 3a verwendete doppelwandigen Drainagefolie 3b aus einem wenig dehnbaren Material besteht, gesteigert werden. Eine schlauchförmige Drainagefolie 3a mit elastischem Material kann durch Verbinden einer doppelwandigen Drainagefolie 3b und eines elastischen Materials längs eines Überlappungsbereichs zwischen der Drainagefolie 3b und dem elastischen Material gebildet werden. Durch das Integrieren des elastischen Materials, beispielsweise eines Streifens aus elastischer Folie 24 oder einer elastischen Naht 25, wird die schlauchförmige Drainagefolie 3a besonders gut aufdehnbar, wodurch die Anwendung, beispielsweise ein Aufbringen auf einen Drainageschlauch, erleichtert wird.

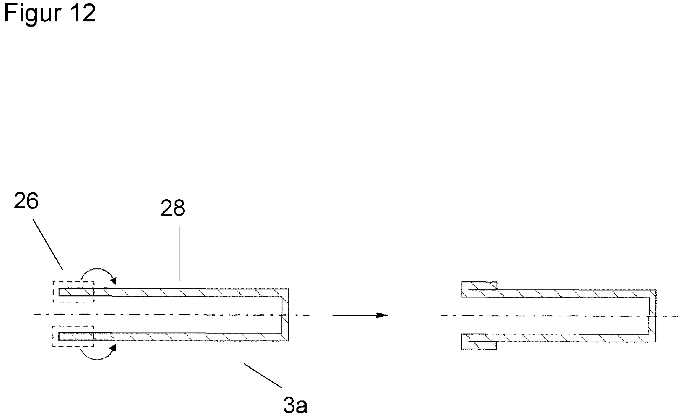

In Fig. 12 ist eine Seitenansicht einer schlauchförmigen Drainagefolie 3a mit durch Umklappen verstärktem Rand dargestellt. Die schlauchförmige Drainagefolie 3a kann an einem oder beiden Schlauchenden in einer Umfangsrichtung mit einem Verstärkungsbereich ausgeführt werden. Herstellungstechnisch hat es sich als besonders effizient erwiesen, wenn der Verstärkungsbereich durch ein Umklappen eines Abschnitts der schlauchförmigen Drainagefolie 3a am Schlauchende gebildet wird, wobei das Umklappen insbesondere derart durchgeführt wird, dass ein die innere Begrenzungsfläche der schlauchförmigen Drainagefolie 3a bildendes schlauchförmiges Element im Verstärkungsbereich die äußere Begrenzungsfläche 28 der schlauchförmigen Drainagefolie 3a bildet. Der gekennzeichnete Randbereich 26 der Schlauchöffnung kann nach außen umgeklappt werden, um so einen doppelwandigen und damit verstärkten Rand der schlauchförmigen Drainagefolie 3a bereitzustellen. Punktuell kann der umgeklappte Rand fixiert werden, beispielsweise durch eine Naht. Ein Einreißen der schlauchförmigen Drainagefolie 3a wird durch den verstärkten Rand effektiv verhindert.

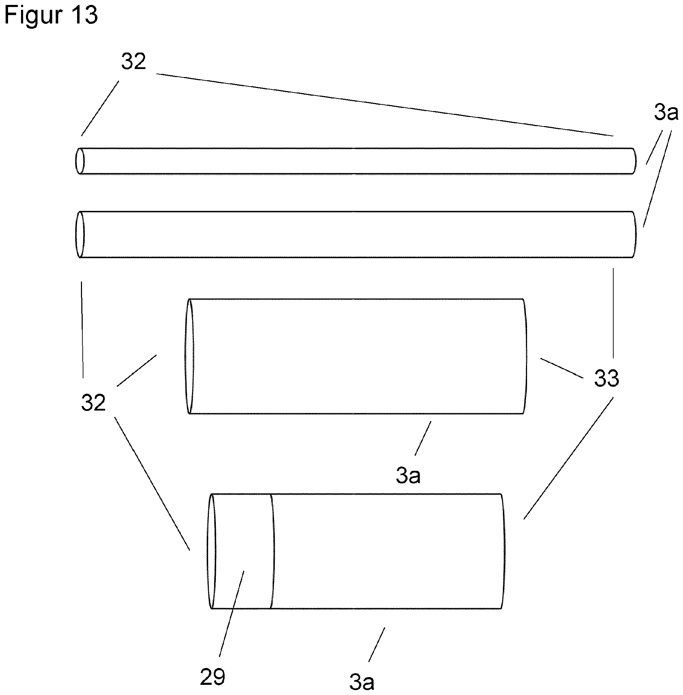

In Fig. 13 sind verschiedene Ausführungen der erfindungsgemäßen schlauchförmigen Drainagefolie 3a dargestellt. Insbesondere kann die schlauchförmige Drainagefolie 3a an einem Schlauchende eine Schlauchöffnung 32 bzw. ein offenes Schlauchende aufweisen und an dem anderen Schlauchende ein geschlossenes Schlauchende 33 aufweisen. Die schlauchförmige Drainagefolie 3a kann auch an beiden Schlauchenden eine Schlauchöffnung 32 aufweisen. Die schlauchförmige Drainagefolie 3a kann darüber hinaus ein Verstärkungselement 29, beispielsweise einen umgeklappten Rand, aufweisen.

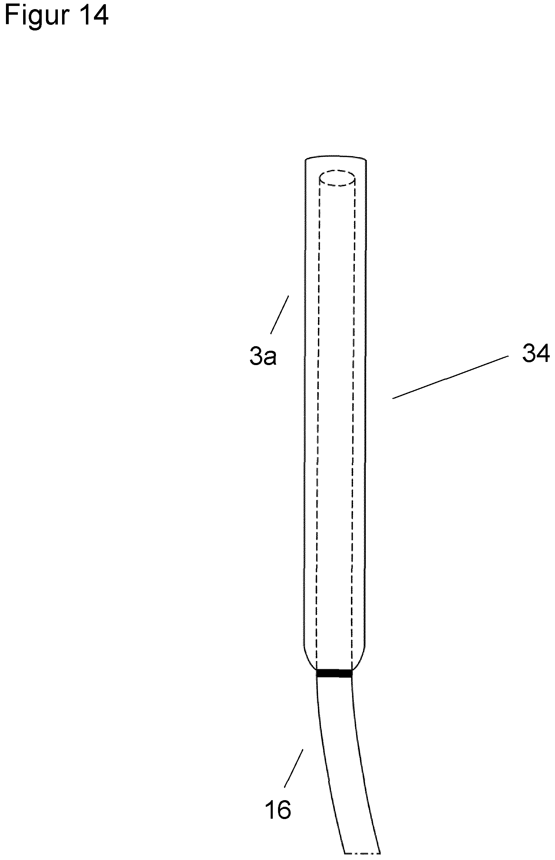

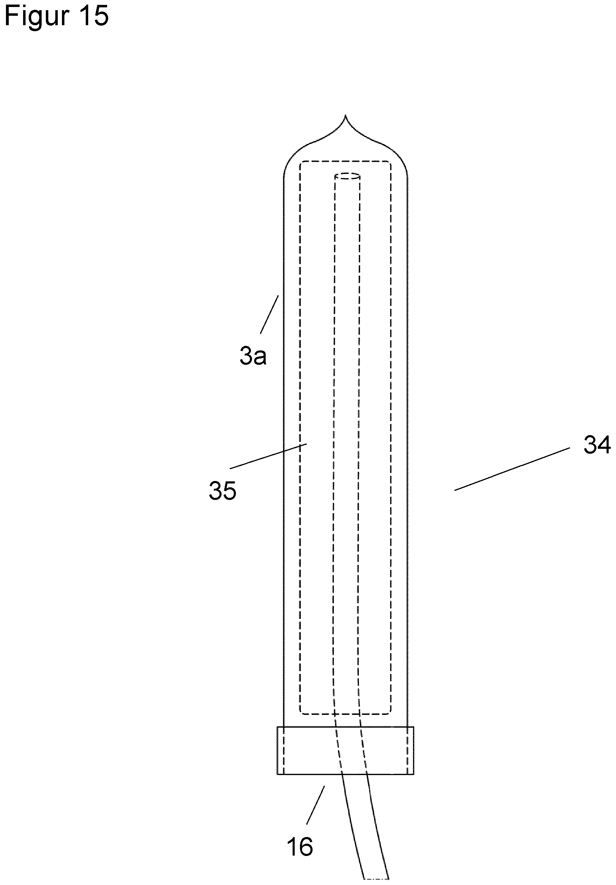

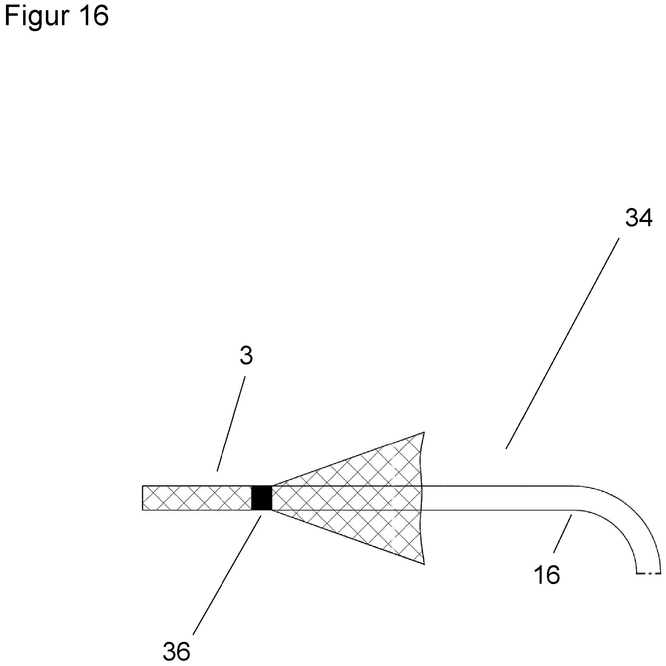

Fig. 14 zeigt eine Seitenansicht einer schlauchförmigen Drainagefolie 3a mit Schlauch 16, die zusammen als Wundversorgungskit 34 bereitgestellt sein können. Fig. 15 zeigt eine Seitenansicht einer schlauchförmigen Drainagefolie 3a mit Schlauch 16 und Absorptionskörper 35, die zusammen als Wundversorgungskit 34 bereitgestellt sein können. Fig. 16 zeigt eine Seitenansicht einer Drainagefolie 3, beispielsweise einer schlauchförmigen Drainagefolie 3a, mit Schlauch 16 und Verbindungsmittel 36, beispielsweise einer ringförmigen Applikationshilfe oder Klammer, die zusammen als Wundversorgungskit 34 bereitgestellt sein können.

### Bezugszeichenliste

- 1: Schweißform
- 2: Bügel
- 3: Drainagefolie
- 3a: schlauchförmige Drainagefolie
- 3b: doppelwandige Drainagefolie
- 4: Fundament
- 5: Schneidelement
- 6: Schweißnaht
- 7: Folienrest
- 8: Stößel
- 9: Hülse
- 10: der Klapp-, insbesondere Faltlinie abgewandte Seite der Verbindungslinie
- 11: Verbindungslinie
- 12: der Klapp-, insbesondere Faltlinie zugewandte Seite der Verbindungslinie
- 13: Gegenstößel
- 14: Haftmittel
- 15: Klettverbindungsmittel
- 15a, 15b: Klettverschluss
- 16: Schlauch
- 17: erstes Element
- 18: zweites Element
- 19: Schneidrad
- 20: Drainageraum
- 21: restliches Material
- 22: Einführhilfe
- 23: Sollbruchstelle
- 24: Elastische Folie
- 25: Elastische Naht
- 26: Randbereich
- 27: Wickelachse
- 28: Äußere Begrenzungsfläche der schlauchförmigen Drainagefolie
- 29: Verstärkungselement
- 30: Schlauchachse
- 31: Innere Begrenzungsfläche der schlauchförmigen Drainagefolie
- 32: Schlauchöffnung
- 33: geschlossenes Schlauchende
- 34: Wundversorgungskit
- 35: Absorptionskörper
- 36: Verbindungsmittel

## Patentansprüche

1. Verfahren zum Herstellen einer doppelwandigen Drainagefolie (3b) mit einem offenen Drainageraum, mit den folgenden Schritten:
i) Bereitstellen eines ersten bahnförmigen Elements und eines zweiten bahnförmigen Elements;
ii) Perforieren des ersten bahnförmigen Elements und des zweiten bahnförmigen Elements, wobei in dem ersten bahnförmigen Element und dem zweiten bahnförmigen Element jeweils mindestens eine den Durchtritt von Körperflüssigkeiten ermöglichende Öffnung, bevorzugt eine Vielzahl von den Durchtritt von Körperflüssigkeiten ermöglichenden Öffnungen, gebildet wird;
iii) Anordnen des ersten bahnförmigen Elements auf dem zweiten bahnförmigen Element derart, dass das erste bahnförmige Element und das zweite bahnförmige Element etwa parallel angeordnet sind;
iv) Verbinden des ersten bahnförmigen Elements und des zweiten bahnförmigen Elements an mindestens einem Befestigungsbereich, insbesondere an mindestens einem punktförmig ausgeführten Befestigungsbereich, bevorzugt durch Ultraschallverschweißen, zum Erhalt einer doppelwandigen Drainagefolie (3b) mit einem zwischen dem ersten bahnförmigen Element und dem zweiten bahnförmigen Element gebildeten offenen Drainageraum,
**dadurch gekennzeichnet, dass** das Verfahren ein Bilden einer schlauchförmigen Struktur aus der Drainagefolie (3, 3a, 3b) umfasst.

2. Verfahren nach Anspruch 1, **dadurch gekennzeichnet, dass** die mindestens eine den Durchtritt von Körperflüssigkeiten ermöglichende Öffnung einen Durchmesser in einem Bereich von 100 µm bis 2000 µm, bevorzugt in einem Bereich von 300 µm bis 700 µm, bevorzugter in einem Bereich von 400 µm bis 600 µm, aufweist, und/oder **dadurch gekennzeichnet, dass** das erste bahnförmige Element und das zweite bahnförmige Element in Schritt iv) an einer Vielzahl von Befestigungsbereichen, bevorzugt an einer Vielzahl von in einer Rasteranordnung ausgebildeten Befestigungsbereichen, verbunden werden;
wobei, bevorzugt, ein Abstand zwischen jeweils benachbarten Befestigungsbereichen 2 mm oder mehr, bevorzugt 3 mm oder mehr, bevorzugter 5 mm oder mehr, beträgt, und/oder **dadurch gekennzeichnet, dass** die mindestens eine den Durchtritt von Körperflüssigkeiten ermöglichende Öffnung, insbesondere bezogen auf eine Aufsicht auf eine flächige Erstreckung des ersten bzw. zweiten bahnförmigen Elements, eine runde, kreisförmige und/oder ovale Form aufweist.

3. Verfahren nach einem der vorangehenden Ansprüche, **dadurch gekennzeichnet, dass** Schritt ii) vor oder nach Schritt iii) durchgeführt wird, und/oder Schritt iv) nach Schritt iii) und vor oder nach Schritt ii) durchgeführt wird.

4. Verfahren zum Herstellen einer schlauchförmigen Drainagefolie (3a), **dadurch gekennzeichnet, dass** das Verfahren die folgenden Schritte umfasst:
a) Bereitstellen einer nach einem der Ansprüche 1 bis 3 hergestellten Drainagefolie (3, 3a, 3b);
b) Umklappen, insbesondere Falten der Drainagefolie (3, 3a, 3b) um eine Klapp-, insbesondere Faltlinie, derart auf sich selbst, dass die Drainagefolie (3, 3a, 3b) einen zu einer Verbindungslinie (11) verbindbaren Überlappungsbereich aufweist; wobei, bevorzugt, die Drainagefolie (3, 3a, 3b) eine erste Kante und eine zur ersten Kante im Wesentlichen parallel verlaufende zweite Kante aufweist und die Drainagefolie (3, 3a, 3b) längs der Klapp- bzw. Faltlinie derart auf sich selbst geklappt bzw. gefaltet wird, dass die Drainagefolie (3, 3a, 3b) entlang der ersten Kante und der zweiten Kante den zu der Verbindungslinie (11) verbindbaren Überlappungsbereich aufweist;
c) Verbinden der Drainagefolie (3, 3a, 3b) längs des Überlappungsbereichs, wobei eine Verbindungslinie (11) gebildet wird;
d) Erhalten einer schlauchförmigen Drainagefolie (3a).

5. Verfahren zum Herstellen einer schlauchförmigen Drainagefolie (3a), **dadurch gekennzeichnet, dass** das Verfahren die folgenden Schritte umfasst:
a) Bereitstellen einer nach einem der Ansprüche 1 bis 3 hergestellten Drainagefolie (3, 3a, 3b), und eines elastischen Materials;
b) Anordnen einer ersten Kante der Drainagefolie (3, 3a, 3b) auf dem elastischen Material derart, dass ein zu einer Verbindungslinie (11) verbindbarer Überlappungsbereich zwischen der ersten Kante der Drainagefolie (3, 3a, 3b) und dem elastischen Material gebildet wird;
c) Verbinden der Drainagefolie (3, 3a, 3b) und des elastischen Materials längs des Überlappungsbereichs zwischen der ersten Kante der Drainagefolie (3, 3a, 3b) und dem elastischen Material, wobei eine Verbindungslinie (11) gebildet wird;
d) Umklappen, insbesondere Falten der Drainagefolie (3, 3a, 3b) bzgl. einer Klapp-, insbesondere einer Faltlinie, derart auf das elastische Material, dass ein zu einer Verbindungslinie (11) verbindbarer Überlappungsbereich zwischen einer zweiten Kante der Drainagefolie (3, 3a, 3b), bevorzugt einer zu der ersten Kante im Wesentlichen parallel verlaufenden zweiten Kante der Drainagefolie (3, 3a, 3b), und dem elastischen Material gebildet wird;
e) Verbinden der Drainagefolie (3, 3a, 3b) und des elastischen Materials längs des Überlappungsbereichs zwischen der zweiten Kante der Drainagefolie (3, 3a, 3b) und dem elastischen Material, wobei eine Verbindungslinie (11) gebildet wird;
f) Erhalten einer schlauchförmigen Drainagefolie (3a).

6. Verfahren zum Herstellen einer schlauchförmigen Drainagefolie (3a), **dadurch gekennzeichnet, dass** das Verfahren die folgenden Schritte umfasst:
a) Bereitstellen einer nach einem der Ansprüche 1 bis 3 hergestellten Drainagefolie (3, 3a, 3b);
b) Wickeln der Drainagefolie (3, 3a, 3b) um eine Wickelachse (8, 27) derart, dass die Drainagefolie (3, 3a, 3b) einen durch das Wickeln um die Wickelachse (8, 27) gebildeten, zu einer Verbindungslinie (11) verbindbaren Überlappungsbereich aufweist, in dem die Drainagefolie (3, 3a, 3b) mindestens doppellagig vorliegt;
c) Verbinden einer ersten Lage und einer zweiten Lage der in dem Überlappungsbereich mindestens doppellagig vorliegenden Drainagefolie (3, 3a, 3b), bevorzugt durch ein Haftmittel (14) oder ein Verbindungsmittel (36); wobei, bevorzugt, eine Verbindungslinie (11) gebildet wird;
d) Erhalten einer schlauchförmigen Drainagefolie (3a).

7. Verfahren nach einem der Ansprüche 4 - 6, **dadurch gekennzeichnet, dass** das Verbinden durch Schweißen, insbesondere Ultraschallverschweißen oder Laserschweißen, durch thermisches Verbinden, insbesondere mittels UV oder Heißluft, durch ein Haftmittel (14), insbesondere einen Klebstoff oder ein Klettverbindungsmittel (15, 15a, 15b), und/oder durch ein Verbindungsmittel, insbesondere eine Klammer, durchgeführt wird.

8. Verfahren nach einem der Ansprüche 4 - 7, **dadurch gekennzeichnet, dass** das Verbinden umfasst: Anordnen der Drainagefolie (3, 3a, 3b) in einer Ultraschallverschweißungsvorrichtung und Verschweißen der Drainagefolie (3, 3a, 3b), insbesondere mittels einer Schweißform (1), z.B. einer L-förmigen Schweißform, zu einer schlauchförmigen Drainagefolie (3a).

9. Verfahren nach einem der Ansprüche 4 - 6 und 7 - 8, **dadurch gekennzeichnet, dass** ein auf einer der Klapp-, insbesondere Faltlinie abgewandten Seite der Verbindungslinie (11) angeordneter Randstreifen der Drainagefolie (3, 3a, 3b) während des Verbindens oder nach dem Verbinden, insbesondere durch Stanzen, entfernt wird.

10. Verfahren nach einem der Ansprüche 4 - 9, **dadurch gekennzeichnet, dass** das Verfahren ein Anbringen eines Verstärkungselements (0, 29), insbesondere eines Verstärkungsstreifens oder einer Verstärkungsfolie, in einem Bereich der Verbindungslinie (11), bevorzugt durch Anordnen des Verstärkungselements (0, 29) im Überlappungsbereich vor dem Verbinden, und/oder an mindestens einem Schlauchende (4, 33), umfasst, vorzugsweise **dadurch gekennzeichnet, dass** das Verstärkungselement (0, 29) eine Folie, bevorzugt eine Polyethylen-Folie (24), ist.

11. Verfahren nach einem der Ansprüche 4 - 10, **dadurch gekennzeichnet, dass** die schlauchförmige Drainagefolie (3a) an mindestens einem Schlauchende (4, 33) in einer Umfangsrichtung mit einem Verstärkungsbereich, in dem die Drainagefolie (3, 3a, 3b) mindestens doppellagig vorliegt, ausgeführt wird;
wobei der Verstärkungsbereich bevorzugt durch ein Umklappen eines Abschnitts der schlauchförmigen Drainagefolie (3a) am Schlauchende (4, 33) gebildet wird, wobei das Umklappen insbesondere derart durchgeführt wird, dass ein die innere Begrenzungsfläche bildendes schlauchförmiges Element im Verstärkungsbereich die äußere Begrenzungsfläche (28) der schlauchförmigen Drainagefolie (3a) bildet.

12. Verfahren nach einem der Ansprüche 4 - 11, **dadurch gekennzeichnet, dass** mindestens ein Abschnitt der schlauchförmigen Drainagefolie (3a) ausgehend von einer Schlauchöffnung (32) an einem Schlauchende (4, 33) entlang einer longitudinalen Schlauchachse (30) aufgerollt wird, insbesondere entlang einer longitudinalen Schlauchachse (30) ausgehend von einer inneren Begrenzungsfläche in Richtung einer äußeren Begrenzungsfläche (28) aufgerollt wird.

13. Verfahren nach einem der Ansprüche 4 - 5 und 7 - 12, **dadurch gekennzeichnet, dass** nach dem Verbinden eine der Klapp-, insbesondere Faltlinie zugewandte Seite der Verbindungslinie (11) durch Umstülpen der schlauchförmigen Drainagefolie (3a) vollständig nach außen gedreht wird.

14. Verfahren nach einem der Ansprüche 4 - 13, **dadurch gekennzeichnet, dass** die in Schritt a) bereitgestellte Drainagefolie (3, 3a, 3b) eine nach einem der Ansprüche 1 bis 5 hergestellte Drainagefolie (3, 3a, 3b) ist.

15. Schlauchförmige Drainagefolie (3a) mit einem eine äußere Begrenzungsfläche (28) bildenden ersten schlauchförmigen Element und einem eine der äußeren Begrenzungsfläche (28) abgewandte innere Begrenzungsfläche bildenden und mit dem ersten schlauchförmigen Element durch mindestens einen Befestigungsbereich verbundenen zweiten schlauchförmigen Element; wobei das erste schlauchförmige Element und das zweite schlauchförmige Element jeweils mindestens eine den Durchtritt von Körperflüssigkeiten ermöglichende Öffnung umfassen und zwischen der äußeren Begrenzungsfläche (28) und der inneren Begrenzungsfläche ein offener Drainageraum gebildet ist.

16. Schlauchförmige Drainagefolie (3a) nach Anspruch 15, **dadurch gekennzeichnet, dass** das erste schlauchförmige Element und/oder das zweite schlauchförmige Element eine Kunststofffolie sind/ist; wobei die Kunststofffolie bevorzugt Polyethylen, Polypropylen, Polyethylenterephthalat, Polyurethan, Polytetrafluorethylen, Polyhydroxybutyrat, Polylactid und/oder Cellulose umfasst.

17. Schlauchförmige Drainagefolie (3a) nach Anspruch 15 oder 16, **dadurch gekennzeichnet, dass** das erste schlauchförmige Element und das zweite schlauchförmige Element an einer Vielzahl von Befestigungsbereichen, bevorzugt an einer Vielzahl von in einer Rasteranordnung ausgebildeten Befestigungsbereichen, verbunden sind; wobei, bevorzugt, ein Abstand zwischen benachbarten Befestigungsbereichen 2 mm oder mehr, bevorzugt 3 mm oder mehr, bevorzugter 5 mm oder mehr, beträgt.

18. Schlauchförmige Drainagefolie (3a) nach einem der Ansprüche 15 bis 17, **dadurch gekennzeichnet, dass** die schlauchförmige Drainagefolie (3a) eine Verbindungslinie (11), bevorzugt eine Schweißnaht (6), aufweist, welche das erste schlauchförmige Element und das zweite schlauchförmige Element, und/oder das erste und/oder zweite schlauchförmige Element und ein elastisches Material, verbindet, vorzugsweise **dadurch gekennzeichnet, dass** die Verbindungslinie (11) entlang einer Umfangsrichtung der schlauchförmigen Drainagefolie (3a) eine Breite von 2 mm oder weniger, bevorzugt 1 mm oder weniger, bevorzugter 0,5 mm oder weniger, aufweist.

19. Schlauchförmige Drainagefolie (3a) nach Anspruch 18, **dadurch gekennzeichnet, dass** die Verbindungslinie (11) entlang einer Radialrichtung der schlauchförmigen Drainagefolie (3a) eine Dicke von 1 mm oder weniger, bevorzugt 750 µm oder weniger, bevorzugter 450 µm oder weniger, aufweist.

20. Schlauchförmige Drainagefolie (3a) nach einem der Ansprüche 15 - 19, **dadurch gekennzeichnet, dass** die schlauchförmige Drainagefolie (3a) ein Flächengewicht in einem Bereich von 10 g/m² bis 200 g/m², bevorzugt 40 g/m² bis 80 g/m², bevorzugter 50 g/m² bis 70 g/m², aufweist.

21. Schlauchförmige Drainagefolie (3a) nach einem der Ansprüche 15 - 20, **dadurch gekennzeichnet, dass** die schlauchförmige Drainagefolie (3a) eine Zugfestigkeit von 7 N/25,4mm oder mehr, eine Reißdehnung von 40% oder mehr, eine Porosität von 75 m³/m²/min oder mehr, und/oder eine Verbundfestigkeit von 0,5 N/25,4mm oder mehr, aufweist.

22. Schlauchförmige Drainagefolie (3a) nach einem der Ansprüche 15 - 21, **dadurch gekennzeichnet, dass** die schlauchförmige Drainagefolie (3a) an mindestens einem Schlauchende (4, 33) eine Schlauchöffnung (32) aufweist; wobei, bevorzugt, die schlauchförmige Drainagefolie (3a) an einem Schlauchende (4, 33) eine Schlauchöffnung (32) und an einem anderen Schlauchende keine Schlauchöffnung (32) aufweist.

23. Schlauchförmige Drainagefolie (3a) nach einem der Ansprüche 15 - 22, **dadurch gekennzeichnet, dass** die schlauchförmige Drainagefolie (3a) an mindestens einem Schlauchende (4, 33) und/oder entlang der Verbindungslinie (11) ein Verstärkungselement (0, 29), insbesondere einen Verstärkungsstreifen oder eine Verstärkungsfolie, aufweist.

24. Schlauchförmige Drainagefolie (3a) nach einem der Ansprüche 15 - 23, **dadurch gekennzeichnet, dass** die schlauchförmige Drainagefolie (3a) an mindestens einem Schlauchende (4, 33) einen Verstärkungsbereich aufweist, in dem die Drainagefolie (3, 3a, 3b) mindestens doppellagig vorliegt; wobei der Verstärkungsbereich bevorzugt in Form eines doppellagigen Randes ausgeführt ist, der durch ein Umklappen eines Abschnitts der schlauchförmigen Drainagefolie (3a) am Schlauchende (4, 33) gebildet ist, wobei das Umklappen insbesondere derart durchgeführt ist, dass das die innere Begrenzungsfläche bildende zweite schlauchförmige Element im Verstärkungsbereich die äußere Begrenzungsfläche (28) der schlauchförmigen Drainagefolie (3a), insbesondere die äußere Begrenzungsfläche (28) des Verstärkungsbereichs der schlauchförmigen Drainagefolie (3a), bildet.

25. Schlauchförmige Drainagefolie (3a) nach einem der Ansprüche 15 - 24, **dadurch gekennzeichnet, dass** die äußere Begrenzungsfläche (28), die innere Begrenzungsfläche und/oder ein von der inneren Begrenzungsfläche umfasstes Innenlumen der schlauchförmigen Drainagefolie (3a) eine Gleithilfe und/oder eine Einführhilfe (22) umfassen/umfasst, vorzugsweise **dadurch gekennzeichnet, dass** die Gleithilfe ein wasserbasiertes Gleitmittel, ein glycerolbasiertes Gleitmittel, ein polymeres Gel, insbesondere ein antiallergenes polymeres Gel, ein endoskopisches Gleitmittel, insbesondere ein endoskopisches Gleitgel, und/oder eine wässrige Lösung, insbesondere eine Natriumchlorid-Lösung, umfasst.

26. Schlauchförmige Drainagefolie (3a) nach Anspruche 25, **dadurch gekennzeichnet, dass** die Einführhilfe (22) ein Textil und/oder einen Kunststoff umfasst; und/oder dass die Einführhilfe (22) ein in dem Innenlumen der schlauchförmigen Drainagefolie (3a) angeordnete schlauchförmige Einführhilfe (22) ist.

27. Wundversorgungskit mit einer nach einem der Ansprüche 4 bis 15 hergestellten schlauchförmigen Drainagefolie (3a) oder einer schlauchförmigen Drainagefolie (3a) nach einem der Ansprüche 17 bis 27, und mindestens einem weiteren Bestandteil, ausgewählt aus einem Absorptionskörper (35), einem Schlauch (7, 16), einem Draht, einem Stent und einer Einführhilfe (22);
wobei, bevorzugt, mindestens ein Abschnitt des mindestens einen weiteren Bestandteils in einem von einer inneren Begrenzungsfläche umfassten Innenlumen der schlauchförmigen Drainagefolie (3a) angeordnet ist.

## Claims

1. Method for producing a double-walled drainage film (3b) with an open drainage space, comprising the following steps:
i) providing a first web-shaped element and a second web-shaped element;
ii) perforating the first web-shaped element and the second web-shaped element, wherein at least one opening allowing the passage of bodily fluids, preferably a plurality of openings allowing the passage of bodily fluids, is formed in each of the first web-shaped element and the second web-shaped element;
iii) arranging the first web-shaped element on the second web-shaped element such that the first web-shaped element and the second web-shaped element are arranged approximately parallel;
iv) connecting the first web-shaped element and the second web-shaped element at at least one fastening region, in particular at at least one punctiform fastening region, preferably by ultrasonic welding, to obtain a double-walled drainage film (3b) with an open drainage space formed between the first web-shaped element and the second web-shaped element,
**characterized in that** the method comprises forming a tubular structure from the drainage film (3, 3a, 3b).

2. Method according to Claim 1, **characterized in that** the at least one opening allowing the passage of bodily fluids has a diameter in a range from 100 µm to 2000 µm, preferably in a range from 300 µm to 700 µm, more preferably in a range from 400 µm to 600 µm, and/or **characterized in that** the first web-shaped element and the second web-shaped element are connected in step iv) at a plurality of fastening regions, preferably at a plurality of fastening regions formed in a grid arrangement;
wherein, preferably, a distance between in each case adjacent fastening regions is 2 mm or more, preferably 3 mm or more, more preferably 5 mm or more, and/or **characterized in that** the at least one opening allowing the passage of bodily fluids has a round, circular and/or oval shape, in particular in relation to a plan view of a planar extent of the first or second web-shaped element.

3. Method according to one of the preceding claims, **characterized in that** step ii) is carried out before or after step iii), and/or step iv) is carried out after step iii) and before or after step ii).

4. Method for producing a tubular drainage film (3a), **characterized in that** the method comprises the following steps:
a) providing a drainage film (3, 3a, 3b) produced according to one of Claims 1 to 3;
b) turning, in particular folding, the drainage film (3, 3a, 3b) onto itself about a turning line, in particular folding line, such that the drainage film (3, 3a, 3b) has an overlapping region which can be connected to form a connecting line (11); wherein, preferably, the drainage film (3, 3a, 3b) has a first edge and a second edge running substantially parallel to the first edge, and the drainage film (3, 3a, 3b) is turned or folded onto itself along the turning or folding line such that the drainage film (3, 3a, 3b) has the overlapping region which can be connected to form the connecting line (11) along the first edge and the second edge;
c) connecting the drainage film (3, 3a, 3b) along the overlapping region, wherein a connecting line (11) is formed;
d) obtaining a tubular drainage film (3a).

5. Method for producing a tubular drainage film (3a), **characterized in that** the method comprises the following steps:
a) providing a drainage film (3, 3a, 3b) produced according to one of Claims 1 to 3, and an elastic material;
b) arranging a first edge of the drainage film (3, 3a, 3b) on the elastic material such that an overlapping region which can be connected to form a connecting line (11) is formed between the first edge of the drainage film (3, 3a, 3b) and the elastic material;
c) connecting the drainage film (3, 3a, 3b) and the elastic material along the overlapping region between the first edge of the drainage film (3, 3a, 3b) and the elastic material, wherein a connecting line (11) is formed;
d) turning, in particular folding, the drainage film (3, 3a, 3b) onto the elastic material with respect to a turning line, in particular a folding line, such that an overlapping region which can be connected to form a connecting line (11) is formed between a second edge of the drainage film (3, 3a, 3b), preferably a second edge of the drainage film (3, 3a, 3b) running substantially parallel to the first edge, and the elastic material;
e) connecting the drainage film (3, 3a, 3b) and the elastic material along the overlapping region between the second edge of the drainage film (3, 3a, 3b) and the elastic material, wherein a connecting line (11) is formed;
f) obtaining a tubular drainage film (3a).

6. Method for producing a tubular drainage film (3a), **characterized in that** the method comprises the following steps:
a) providing a drainage film (3, 3a, 3b) produced according to one of Claims 1 to 3;
b) winding the drainage film (3, 3a, 3b) about a winding axis (8, 27) such that the drainage film (3, 3a, 3b) has an overlapping region which is formed by the winding about the winding axis (8, 27) and can be connected to form a connecting line (11) and in which the drainage film (3, 3a, 3b) is at least double-layered;
c) connecting a first layer and a second layer of the drainage film (3, 3a, 3b) which is at least double-layered in the overlapping region, preferably by an adhesive (14) or a connecting means (36); wherein, preferably, a connecting line (11) is formed;
d) obtaining a tubular drainage film (3a).

7. Method according to one of Claims 4 - 6, **characterized in that** the connection is carried out by welding, in particular ultrasonic welding or laser welding, by thermal connection, in particular by means of UV or hot air, by an adhesive (14), in particular an adhesive or a Velcro connection means (15, 15a, 15b), and/or by a connecting means, in particular a clamp.

8. Method according to one of Claims 4 - 7, **characterized in that** the connection comprises: arranging the drainage film (3, 3a, 3b) in an ultrasonic welding device and welding the drainage film (3, 3a, 3b), in particular by means of a welding die (1), for example an L-shaped welding die, to form a tubular drainage film (3a).

9. Method according to one of Claims 4 - 6 and 7 - 8, **characterized in that** an edge strip of the drainage film (3, 3a, 3b) arranged on a side of the connecting line (11) facing away from the turning line, in particular folding line, is removed during the connection or after the connection, in particular by punching.

10. Method according to one of Claims 4 - 9, **characterized in that** the method comprises attaching a reinforcing element (0, 29), in particular a reinforcing strip or a reinforcing film, in a region of the connecting line (11), preferably by arranging the reinforcing element (0, 29) in the overlapping region before the connection, and/or at at least one tube end (4, 33), preferably **characterized in that** the reinforcing element (0, 29) is a film, preferably a polyethylene film (24).

11. Method according to one of Claims 4 - 10, **characterized in that** the tubular drainage film (3a) is formed at at least one tube end (4, 33) in a circumferential direction with a reinforcing region in which the drainage film (3, 3a, 3b) is at least double-layered;
wherein the reinforcing region is preferably formed by turning a section of the tubular drainage film (3a) at the tube end (4, 33), wherein the turning is carried out in particular such that a tubular element forming the inner boundary surface forms the outer boundary surface (28) of the tubular drainage film (3a) in the reinforcing region.

12. Method according to one of Claims 4 - 11, **characterized in that** at least one section of the tubular drainage film (3a) is rolled up starting from a tube opening (32) at a tube end (4, 33) along a longitudinal tube axis (30), in particular is rolled up along a longitudinal tube axis (30) starting from an inner boundary surface in the direction of an outer boundary surface (28).

13. Method according to one of Claims 4 - 5 and 7 - 12, **characterized in that**, after the connection, a side of the connecting line (11) facing the turning line, in particular folding line, is rotated completely outwards by turning the tubular drainage film (3a).

14. Method according to one of Claims 4 - 13, **characterized in that** the drainage film (3, 3a, 3b) provided in step a) is a drainage film (3, 3a, 3b) produced according to one of Claims 1 to 5.

15. Tubular drainage film (3a) comprising a first tubular element forming an outer boundary surface (28) and a second tubular element forming an inner boundary surface facing away from the outer boundary surface (28) and connected to the first tubular element by at least one fastening region; wherein the first tubular element and the second tubular element each comprise at least one opening allowing the passage of bodily fluids and an open drainage space is formed between the outer boundary surface (28) and the inner boundary surface.

16. Tubular drainage film (3a) according to Claim 15, **characterized in that** the first tubular element and/or the second tubular element is/are a plastics film; wherein the plastics film preferably comprises polyethylene, polypropylene, polyethylene terephthalate, polyurethane, polytetrafluoroethylene, polyhydroxybutyrate, polylactide and/or cellulose.

17. Tubular drainage film (3a) according to Claim 15 or 16, **characterized in that** the first tubular element and the second tubular element are connected at a plurality of fastening regions, preferably at a plurality of fastening regions formed in a grid arrangement; wherein, preferably, a distance between adjacent fastening regions is 2 mm or more, preferably 3 mm or more, more preferably 5 mm or more.

18. Tubular drainage film (3a) according to one of Claims 15 to 17, **characterized in that** the tubular drainage film (3a) has a connecting line (11), preferably a weld seam (6), which connects the first tubular element and the second tubular element, and/or the first and/or second tubular element and an elastic material, preferably **characterized in that** the connecting line (11) has a width of 2 mm or less, preferably 1 mm or less, more preferably 0.5 mm or less, along a circumferential direction of the tubular drainage film (3a).

19. Tubular drainage film (3a) according to Claim 18, **characterized in that** the connecting line (11) has a thickness of 1 mm or less, preferably 750 µm or less, more preferably 450 µm or less, along a radial direction of the tubular drainage film (3a).

20. Tubular drainage film (3a) according to one of Claims 15 - 19, **characterized in that** the tubular drainage film (3a) has a basis weight in a range from 10 g/m2 to 200 g/m², preferably 40 g/m2 to 80 g/m2, more preferably 50 g/m2 to 70 g/m2.

21. Tubular drainage film (3a) according to one of Claims 15 - 20, **characterized in that** the tubular drainage film (3a) has a tensile strength of 7 N/25.4 mm or more, an elongation at break of 40% or more, a porosity of 75 m3/m2/min or more, and/or a bond strength of 0.5 N/25.4 mm or more.

22. Tubular drainage film (3a) according to one of Claims 15 - 21, **characterized in that** the tubular drainage film (3a) has a tube opening (32) at at least one tube end (4, 33); wherein, preferably, the tubular drainage film (3a) has a tube opening (32) at one tube end (4, 33) and no tube opening (32) at another tube end.

23. Tubular drainage film (3a) according to one of Claims 15 - 22, **characterized in that** the tubular drainage film (3a) has a reinforcing element (0, 29), in particular a reinforcing strip or a reinforcing film, at at least one tube end (4, 33) and/or along the connecting line (11).

24. Tubular drainage film (3a) according to one of Claims 15 - 23, **characterized in that** the tubular drainage film (3a) has a reinforcing region at at least one tube end (4, 33) in which the drainage film (3, 3a, 3b) is at least double-layered; wherein the reinforcing region is preferably formed in the form of a double-layered edge which is formed by turning a section of the tubular drainage film (3a) at the tube end (4, 33), wherein the turning is carried out in particular such that the second tubular element forming the inner boundary surface forms the outer boundary surface (28) of the tubular drainage film (3a), in particular the outer boundary surface (28) of the reinforcing region of the tubular drainage film (3a), in the reinforcing region.

25. Tubular drainage film (3a) according to one of Claims 15 - 24, **characterized in that** the outer boundary surface (28), the inner boundary surface and/or an inner lumen of the tubular drainage film (3a) comprised by the inner boundary surface comprise/comprises a sliding aid and/or an insertion aid (22), preferably **characterized in that** the sliding aid comprises a waterbased lubricant, a glycerol-based lubricant, a polymeric gel, in particular an antiallergenic polymeric gel, an endoscopic lubricant, in particular an endoscopic sliding gel, and/or an aqueous solution, in particular a sodium chloride solution.

26. Tubular drainage film (3a) according to Claim 25, **characterized in that** the insertion aid (22) comprises a textile and/or a plastic; and/or **in that** the insertion aid (22) is a tubular insertion aid (22) arranged in the inner lumen of the tubular drainage film (3a).

27. Wound care kit comprising a tubular drainage film (3a) produced according to one of Claims 4 to 15 or a tubular drainage film (3a) according to one of Claims 17 to 27, and at least one further constituent, selected from an absorption body (35), a tube (7, 16), a wire, a stent and an insertion aid (22);
wherein, preferably, at least one section of the at least one further constituent is arranged in an inner lumen, comprised by an inner boundary surface, of the tubular drainage film (3a).

## Revendications

1. Procédé de fabrication d'une feuille de drainage à double paroi (3b) avec un espace de drainage ouvert, comprenant les étapes suivantes :
i) Fourniture d'un premier élément en forme de bande et d'un second élément en forme de bande ; ii) Perforation du premier élément en forme de bande et du second élément en forme de bande, dans lesquels au moins une ouverture permettant le passage de liquides corporels, de préférence une pluralité d'ouvertures permettant le passage de liquides corporels, est formée dans le premier élément en forme de bande et dans le second élément en forme de bande respectivement ;
iii) Disposition du premier élément en forme de bande sur le second élément en forme de bande de telle sorte que le premier élément en forme de bande et le second élément en forme de bande sont disposés de manière sensiblement parallèle ;
iv) Liaison du premier élément en forme de bande et du second élément en forme de bande au moins dans une zone de fixation, en particulier dans au moins une zone de fixation ponctuelle, de préférence par soudage par ultrasons, pour obtenir une feuille de drainage à double paroi (3b) avec un espace de drainage ouvert formé entre le premier élément en forme de bande et le second élément en forme de bande,
**caractérisé en ce que** le procédé comprend la formation d'une structure en forme de tube à partir de la feuille de drainage (3, 3a, 3b).

2. Procédé selon la revendication 1, **caractérisé en ce que** l'au moins une ouverture permettant le passage de liquides corporels présente un diamètre dans une plage de 100 µm à 2000 µm, de préférence dans une plage de 300 µm à 700 µm, plus préférentiellement dans une plage de 400 µm à 600 µm, et/ou **caractérisé en ce que** le premier élément en forme de bande et le second élément en forme de bande dans l'étape iv) sont liés dans une pluralité de zones de fixation, de préférence dans une pluralité de zones de fixation formées selon un agencement en grille ;
où, de préférence, une distance entre des zones de fixation adjacentes est de 2 mm ou plus, de préférence 3 mm ou plus, plus préférentiellement 5 mm ou plus, et/ou **caractérisé en ce que** l'au moins une ouverture permettant le passage de liquides corporels, en particulier par rapport à une vue en plan d'une extension surfacique du premier ou du second élément en forme de bande, présente une forme ronde, circulaire et/ou ovale.

3. Procédé selon l'une des revendications précédentes, **caractérisé en ce que** l'étape ii) est réalisée avant ou après l'étape iii), et/ou l'étape iv) est réalisée après l'étape iii) et avant ou après l'étape ii).

4. Procédé de fabrication d'une feuille de drainage en forme de tube (3a), **caractérisé en ce que** le procédé comprend les étapes suivantes :
a) Fourniture d'une feuille de drainage (3, 3a, 3b) fabriquée selon l'une des revendications 1 à 3 ;
b) Rabattement, en particulier pliage de la feuille de drainage (3, 3a, 3b) autour d'une ligne de pliage, en particulier ligne de pliage, sur elle-même de telle sorte que la feuille de drainage (3, 3a, 3b) présente une zone de recouvrement pouvant être liée à une ligne de liaison (11) ; où, de préférence, la feuille de drainage (3, 3a, 3b) présente un premier bord et un second bord s'étendant sensiblement parallèlement au premier bord, et la feuille de drainage (3, 3a, 3b) est rabattue ou pliée sur elle-même le long de la ligne de pliage de telle sorte que la feuille de drainage (3, 3a, 3b) présente le long du premier bord et du second bord la zone de recouvrement pouvant être liée à la ligne de liaison (11) ;
c) Liaison de la feuille de drainage (3, 3a, 3b) le long de la zone de recouvrement, une ligne de liaison (11) étant formée ;
d) Obtention d'une feuille de drainage en forme de tube (3a).

5. Procédé de fabrication d'une feuille de drainage en forme de tube (3a), **caractérisé en ce que** le procédé comprend les étapes suivantes :
a) Fourniture d'une feuille de drainage (3, 3a, 3b) fabriquée selon l'une des revendications 1 à 3, et d'un matériau élastique ; b) Disposition d'un premier bord de la feuille de drainage (3, 3a, 3b) sur le matériau élastique de telle sorte qu'une zone de recouvrement pouvant être liée à une ligne de liaison (11) est formée entre le premier bord de la feuille de drainage (3, 3a, 3b) et le matériau élastique ; c) Liaison de la feuille de drainage (3, 3a, 3b) et du matériau élastique le long de la zone de recouvrement entre le premier bord de la feuille de drainage (3, 3a, 3b) et le matériau élastique, une ligne de liaison (11) étant formée ; d) Rabattement, en particulier pliage de la feuille de drainage (3, 3a, 3b) par rapport à une ligne de pliage, en particulier une ligne de pliage, sur le matériau élastique de telle sorte qu'une zone de recouvrement pouvant être liée à une ligne de liaison (11) est formée entre un second bord de la feuille de drainage (3, 3a, 3b), de préférence un second bord s'étendant sensiblement parallèlement au premier bord de la feuille de drainage (3, 3a, 3b), et le matériau élastique ;
e) Liaison de la feuille de drainage (3, 3a, 3b) et du matériau élastique le long de la zone de recouvrement entre le second bord de la feuille de drainage (3, 3a, 3b) et le matériau élastique, une ligne de liaison (11) étant formée ;
f) Obtention d'une feuille de drainage en forme de tube (3a).

6. Procédé de fabrication d'une feuille de drainage en forme de tube (3a), **caractérisé en ce que** le procédé comprend les étapes suivantes :
a) Fourniture d'une feuille de drainage (3, 3a, 3b) fabriquée selon l'une des revendications 1 à 3 ;
b) Enroulement de la feuille de drainage (3, 3a, 3b) autour d'un axe d'enroulement (8, 27) de telle sorte que la feuille de drainage (3, 3a, 3b) présente une zone de recouvrement pouvant être liée à une ligne de liaison (11) formée par l'enroulement autour de l'axe d'enroulement (8, 27), dans laquelle la feuille de drainage (3, 3a, 3b) est au moins à double couche ;
c) Liaison d'une première couche et d'une seconde couche de la feuille de drainage (3, 3a, 3b) présente au moins à double couche dans la zone de recouvrement, de préférence par un agent adhésif (14) ou un moyen de liaison (36) ; où, de préférence, une ligne de liaison (11) est formée ;
d) Obtention d'une feuille de drainage en forme de tube (3a).

7. Procédé selon l'une des revendications 4 à 6, **caractérisé en ce que** la liaison est réalisée par soudage, en particulier soudage par ultrasons ou soudage laser, par liaison thermique, en particulier au moyen d'UV ou d'air chaud, par un agent adhésif (14), en particulier un adhésif ou un moyen de fixation à crochets et boucles (15, 15a, 15b), et/ou par un moyen de liaison, en particulier une agrafe.

8. Procédé selon l'une des revendications 4 à 7, **caractérisé en ce que** la liaison comprend : disposition de la feuille de drainage (3, 3a, 3b) dans un dispositif de soudage par ultrasons et soudage de la feuille de drainage (3, 3a, 3b), en particulier au moyen d'une matrice de soudage (1), par exemple une matrice de soudage en forme de L, pour obtenir une feuille de drainage en forme de tube (3a).

9. Procédé selon l'une des revendications 4 à 6 et 7 à 8, **caractérisé en ce qu'**une bande marginale de la feuille de drainage (3, 3a, 3b) disposée sur un côté de la ligne de pliage opposé à la ligne de liaison (11) est retirée pendant ou après la liaison, en particulier par découpe.
L31599WO Lohmann & Rauscher GmbH 25.03.2025

10. Procédé selon l'une des revendications 4 à 9, **caractérisé en ce que** le procédé comprend la fixation d'un élément de renforcement (0, 29), en particulier d'une bande de renforcement ou d'un film de renforcement, dans une zone de la ligne de liaison (11), de préférence par disposition de l'élément de renforcement (0, 29) dans la zone de recouvrement avant la liaison, et/ou à au moins une extrémité du tube (4, 33), de préférence **caractérisé en ce que** l'élément de renforcement (0, 29) est un film, de préférence un film de polyéthylène (24).

11. Procédé selon l'une des revendications 4 à 10, **caractérisé en ce que** la feuille de drainage en forme de tube (3a) est réalisée à au moins une extrémité du tube (4, 33) dans une direction circonférentielle avec une zone de renforcement dans laquelle la feuille de drainage (3, 3a, 3b) est au moins à double couche ;
où la zone de renforcement est de préférence formée par rabattement d'une section de la feuille de drainage en forme de tube (3a) à l'extrémité du tube (4, 33), le rabattement étant en particulier réalisé de telle sorte qu'un élément en forme de tube formant la surface limite interne forme la surface limite externe (28) de la feuille de drainage en forme de tube (3a).

12. Procédé selon l'une des revendications 4 à 11, **caractérisé en ce qu'**au moins une section de la feuille de drainage en forme de tube (3a) est enroulée à partir d'une ouverture du tube (32) à une extrémité du tube (4, 33) le long d'un axe longitudinal du tube (30), en particulier le long d'un axe longitudinal du tube (30) à partir d'une surface limite interne vers une surface limite externe (28).

13. Procédé selon l'une des revendications 4 à 5 et 7 à 12, **caractérisé en ce qu'**après la liaison, un côté de la ligne de liaison (11) orienté vers la ligne de pliage est entièrement tourné vers l'extérieur par retournement de la feuille de drainage en forme de tube (3a).

14. Procédé selon l'une des revendications 4 à 13, **caractérisé en ce que** la feuille de drainage (3, 3a, 3b) fournie à l'étape a) est une feuille de drainage (3, 3a, 3b) fabriquée selon l'une des revendications 1 à 5.

15. Feuille de drainage en forme de tube (3a) comprenant un premier élément en forme de tube formant une surface limite externe (28) et un second élément en forme de tube formant une surface limite interne opposée à la surface limite externe (28) et lié au premier élément en forme de tube par au moins une zone de fixation ;
où le premier élément en forme de tube et le second élément en forme de tube comprennent chacun au moins une ouverture permettant le passage de liquides corporels et un espace de drainage ouvert est formé entre la surface limite externe (28) et la surface limite interne.

16. Feuille de drainage en forme de tube (3a) selon la revendication 15, **caractérisée en ce que** le premier élément en forme de tube et/ou le second élément en forme de tube sont/est un film plastique ; où le film plastique comprend de préférence du polyéthylène, du polypropylène, du polyéthylène téréphtalate, du polyuréthane, du polytétrafluoroéthylène, du polyhydroxybutyrate, du polylactide et/ou de la cellulose.

17. Feuille de drainage en forme de tube (3a) selon la revendication 15 ou 16, **caractérisée en ce que** le premier élément en forme de tube et le second élément en forme de tube sont liés dans une pluralité de zones de fixation, de préférence dans une pluralité de zones de fixation formées selon un agencement en grille ; où, de préférence, une distance entre des zones de fixation adjacentes est de 2 mm ou plus, de préférence 3 mm ou plus, plus préférentiellement 5 mm ou plus.

18. Feuille de drainage en forme de tube (3a) selon l'une des revendications 15 à 17, **caractérisée en ce que** la feuille de drainage en forme de tube (3a) présente une ligne de liaison (11), de préférence une soudure (6), qui lie le premier élément en forme de tube et le second élément en forme de tube, et/ou le premier et/ou le second élément en forme de tube et un matériau élastique, de préférence **caractérisée en ce que** la ligne de liaison (11) présente, le long d'une direction circonférentielle de la feuille de drainage en forme de tube (3a), une largeur de 2 mm ou moins, de préférence 1 mm ou moins, plus préférentiellement 0,5 mm ou moins.

19. Feuille de drainage en forme de tube (3a) selon la revendication 18, **caractérisée en ce que** la ligne de liaison (11) présente, le long d'une direction radiale de la feuille de drainage en forme de tube (3a), une épaisseur de 1 mm ou moins, de préférence 750 µm ou moins, plus préférentiellement 450 µm ou moins.

20. Feuille de drainage en forme de tube (3a) selon l'une des revendications 15 à 19, **caractérisée en ce que** la feuille de drainage en forme de tube (3a) présente une masse surfacique dans une plage de 10 g/m² à 200 g/m², de préférence 40 g/m² à 80 g/m², plus préférentiellement 50 g/m² à 70 g/m².

21. Feuille de drainage en forme de tube (3a) selon l'une des revendications 15 à 20, **caractérisée en ce que** la feuille de drainage en forme de tube (3a) présente une résistance à la traction de 7N/25,4mm ou plus, un allongement à la rupture de 40% ou plus, une porosité de 75 m³/m²/min ou plus, et/ou une résistance du composite de 0,5 N/25,4mm ou plus.

22. Feuille de drainage en forme de tube (3a) selon l'une des revendications 15 à 21, **caractérisée en ce que** la feuille de drainage en forme de tube (3a) présente au moins une ouverture du tube (32) à au moins une extrémité du tube (4, 33) ; où, de préférence, la feuille de drainage en forme de tube (3a) présente une ouverture du tube (32) à une extrémité du tube (4, 33) et aucune ouverture du tube (32) à une autre extrémité du tube.

23. Feuille de drainage en forme de tube (3a) selon l'une des revendications 15 à 22, **caractérisée en ce que** la feuille de drainage en forme de tube (3a) présente au moins un élément de renforcement (0, 29), en particulier une bande de renforcement ou un film de renforcement, à au moins une extrémité du tube (4, 33) et/ou le long de la ligne de liaison (11).

24. Feuille de drainage en forme de tube (3a) selon l'une des revendications 15 à 23, **caractérisée en ce que** la feuille de drainage en forme de tube (3a) présente à au moins une extrémité du tube (4, 33) une zone de renforcement dans laquelle la feuille de drainage (3, 3a, 3b) est au moins à double couche ; où la zone de renforcement est de préférence sous la forme d'un bord à double couche formé par rabattement d'une section de la feuille de drainage en forme de tube (3a) à l'extrémité du tube (4, 33), le rabattement étant en particulier réalisé de telle sorte que le second élément en forme de tube formant la surface limite interne forme dans la zone de renforcement la surface limite externe (28) de la feuille de drainage en forme de tube (3a), en particulier la surface limite externe (28) de la zone de renforcement de la feuille de drainage en forme de tube (3a).

25. Feuille de drainage en forme de tube (3a) selon l'une des revendications 15 à 24, **caractérisée en ce que** la surface limite externe (28), la surface limite interne et/ou une lumière interne comprise par la surface limite interne de la feuille de drainage en forme de tube (3a) comprennent/comprend une aide au glissement et/ou une aide à l'introduction (22), de préférence **caractérisée en ce que** l'aide au glissement comprend un lubrifiant à base d'eau, un lubrifiant à base de glycérol, un gel polymère, en particulier un gel polymère anti-allergène, un lubrifiant endoscopique, en particulier un gel lubrifiant endoscopique, et/ou une solution aqueuse, en particulier une solution de chlorure de sodium.

26. Feuille de drainage en forme de tube (3a) selon la revendication 25, **caractérisée en ce que** l'aide à l'introduction (22) comprend un textile et/ou un plastique ; et/ou **en ce que** l'aide à l'introduction (22) est une aide à l'introduction en forme de tube (22) disposée dans la lumière interne de la feuille de drainage en forme de tube (3a).

27. Kit de traitement des plaies comprenant une feuille de drainage en forme de tube (3a) fabriquée selon l'une des revendications 4 à 15 ou une feuille de drainage en forme de tube (3a) selon l'une des revendications 17 à 27, et au moins un autre composant, sélectionné parmi un corps absorbant (35), un tube (7, 16), un fil, un stent et une aide à l'introduction (22) ;
où, de préférence, au moins une section du ou des autres composants est disposée dans une lumière interne comprise par une surface limite interne de la feuille de drainage en forme de tube (3a).
